(19) Europäisches Patentamt
European Patent Office
Office européen des brevets

(11) **EP 1 319 949 A1**

(12) **EUROPEAN PATENT APPLICATION**
published in accordance with Art. 158(3) EPC

(43) Date of publication:
**18.06.2003 Bulletin 2003/25**

(51) Int Cl.⁷: **G01N 33/483**, G01N 33/53,
G01N 27/06

(21) Application number: **01970169.7**

(22) Date of filing: **20.09.2001**

(86) International application number:
**PCT/JP01/08188**

(87) International publication number:
**WO 02/025274 (28.03.2002 Gazette 2002/12)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **20.09.2000 JP 2000286188**

(71) Applicant: **Seino, Yuko**
**Saitama-shi, Saitama 330-0033 (JP)**

(72) Inventors:
• **SEINO, Yuko**
**Saitama-shi, Saitama 330-0033 (JP)**
• **ISHII, Masaru**
**Saitama-shi, Saitama 330-0021 (JP)**
• **GOTSU, Toshio**
**Iruma-shi, Saitama 358-0026 (JP)**

(74) Representative: **Holdcroft, James Gerald, Dr. et al**
**Graham Watt & Co.**
**3 Gray's Inn Square**
**London WC1R 5AH (GB)**

(54) **REACTION DETECTING METHOD, IMMUNOREACTION DETECTING METHOD AND DEVICE**

(57) Reaction of a substance can be detected more simply and quickly without requiring expensive, large-scale equipment and measuring instruments. A reaction detecting method detects a reaction of a substance in an electrolytic solution by measuring an electric conductivity of the electrolytic solution. An immunorcaction detecting method detects an immunorcaction of an antigen and an antibody in an electrolytic solution by measuring an electric conductivity of the electrolytic solution. Another immunoreaction detecting method detects an immunoreaction of an antigen and an antibody in a liquid to be examined by measuring the temperature of the liquid to be examined.

FIG.1A

FIG.1B

**Description**

Technical Field

The present invention relates to a reaction detecting method and an apparatus therefor which permit simple detection of a reaction of substances, or more specifically, reaction products and the status of reaction, and are useful for detection and quantitative determination of specific substances in a sample.

Background Art

A reaction of substances have conventionally been detected by various methods depending upon the type of reaction, the kinds of reactants, and the like, and specific apparatuses and reagents corresponding to the individual objects have been provided. For example, for the purpose of detecting an immunological reaction between an antigen and an antibody, immunological measuring methods have made a progress.

Hereinafter, in the present specification, importance of description is placed on reactions to which biological molecules are related as reactions in an electrolytic solution, including immunological reactions (immune reactions) between an antigen and an antibody, and enzymatic reactions (enzyme reactions) between an enzyme and a biological substrate, but the present invention is not limited thereto.

From the clinical point of view such as inspection and diagnosis of a disease, detection of a reaction based on an immunological or enzymatic specificity is very important. For example, cancer-related substances having a high specificity to various kinds of cancer are known to be present in the body fluid of a cancer patient. More specifically, a so-called tumor marker (tumor-related antigen) typically represented by carcinoembryonic protein is known to excessively express along with canceration of cells, and the amount of the tumor marker is considered to increase with progress of cancer. Cancer-related substances include also genes (cancer-related gene, oncogenes) products considered to be deeply associated with carcinogenesis or progress of cancer, various hormones excessively expressing in a hormone-dependent cancer tissue and receptors thereof.

Detection of trace cancer-related substances having a high specificity to cancer or detection of antibodies against these substances is important at various clinical stages such as cancer diagnosis, determination of a therapeutic indicator and prognostic inspection. A simpler and more rapid detection thereof is very important from the point of view of early detection of cancer.

More particularly, for the purpose of detecting and quantitatively determining cancer-related substances such as a tumor marker or antibodies against these substances, the radio immunoassay method (RIA method) and the enzyme immunoassay method (EIA method) are conventionally used in general. The RIA and EIA methods are immunologically measuring methods which detect an antigen, an antibody or an immune complex by use of the reaction based on the immunological specificity.

As is well known by those skilled in the art, when an antigen substance such as a tumor marker is detected and quantitatively determined by the RIA method or the EIA method, the so-called sandwich method or the competitive method are commonly applied. The sandwich method comprises the steps of, for example, solidifying an antibody (first antibody) specifically reactive with the antigen substance to be detected into a carrier, bringing this solidified antibody (first antibody) into contact with a sample, separating the fraction not having reacted (conjugated) with the solidified antibody (first antibody), then causing a antibody (second antibody) labelled with radioactive substance (RIA method) or enzyme (EIA method) recognizing the same antigen as the solidified antibody (first antibody) to react, and detecting and determining the resultant immune complex by measuring the labelled substance. The competition method comprises the steps of, for example, solidifying an antibody specifically reacting with the antigen substance to be detected into a carrier, and causing a competitive reaction between a labelled antigen or an antibody reactive specifically with this solidified antibody and a sample relative to the solidified antibody. Subsequently, the target antigen in the sample is detected and quantitatively determined by measuring the labelled substance of the resultant immune complex. At all events, the final amount of immune reaction is determined, in the RIA method, from the radioactivity of the labelled radioactive substance, and in the EIA method, by measuring the enzymatic activity of the labelled enzyme. The enzymatic activity is measured, for example, from light emitting intensity caused by the reaction between the enzyme substrate serving as a coloring agent and the enzyme.

In the conventional immunological measuring methods, as described above, it is necessary to provide a solidified carrier such as beads or a plate, a labelled substance such as a radioactive substance or an enzyme, and in the EIA method, an enzyme substance such as a coloring agent.

The above-mentioned conventional immunological measuring methods are popularly applied because of the possibility to detect and quantitatively determine specifically and at a high sensitivity the target substance by detecting specific immunological reactions, respectively.

However, the aforementioned RIA and EIA methods require such procedures as solidification of an antigen or an antibody into a carrier, and preparation of a labelled substance, leading to necessity of complicated operations and much time. In these methods, a special reagent such as a labelled substance or a coloring agent (enzyme substrate) is necessary for each substance to be measured. A special measuring instrument for each labelled substance, for example, a radiation detector for the RIA method, and a fluorescence detector or a light emission detector for in the EIA method in accordance with labelled substances must be provided. These instruments are generally complicated in structure and relatively expensive.

In order to detect individual reactions, it is thus necessary to provide a special reagent and a special measuring instrument for each of various measurements, resulting in a higher cost. These circumstances lead to an increase in the quantity of waste of various reagents or various appliances such as solidification carriers, posing environmental problems.

Measurement of radioactivity in the RIA method, for example, requires a special facility and a special operator, and cannot be carried out easily.

Furthermore, the aforementioned RIA and EIA methods have an object to detect final reaction products which are immune complex, and therefore, it is not easy to observe with time the status of reaction. If it is possible to time-dependently detect the status of reaction by a simple method, there would be available various advantages including the possibility to easily select an antibody more reactive with an antigen or to easily determine presence of sensitivity between antigen and antibody. However, such a method has not as yet been available.

The conventional reaction detecting method and the problems involved therein, particularly with respect to immunological reactions, have been described above. However, requirements for needlessness of large-scaled measuring instruments or special facilities or operator, a smaller amount of waste of reagents, and the possibility to rapidly and simply detect reactions are common to all reactions. Possibility to time-dependently observe the status of reaction of substances by means of instruments of a simple configuration would be useful in various fields of art.

A object of the present invention, in general, is therefore to provide a reaction detecting method and apparatus therefor which permit detection of reactions of substances more easily.

Another object of the invention is to provide a reaction detecting method and an apparatus therefor which do not require expensive and large-scaled facilities or measuring instruments, easy and rapid detection in a real-time manner of the time-dependent reaction status and/or reaction products of reactions of substances, and are applicable, for example, for detection and quantitative determination of a specific substance in a sample.

A still another object of the invention is to provide a reaction detecting method and an apparatus therefor which permit easier detection of an immunological or enzymatic reaction, and make it possible, for example, to detect and quantitatively determine specific substances associated with a specific state of a disease more simply.

A further another object of the invention is to provide a reaction detecting method which give a new approach to detect the time-dependent reaction status and/or reaction products of reactions of various substances taking place in an electrolytic solution simply and rapidly.

An additional object of the invention is to provide an immune reaction measuring method and an apparatus therefor which permit very easy detection of an immunological reaction, and very easy and rapid detection and quantitative determination in a real-time manner of specific substances in a sample such as a specific substance associated with a specific state of a disease.

Disclosure of the Invention

The aforementioned objects of the present invention are achieved by the reaction detecting method, the immune reaction detecting method and the apparatus therefor of the invention. In summary, a first aspect of the invention provides a reaction detecting method comprising detecting a reaction of a substance in an electrolytic solution on the basis of measurement of an electric conductivity of the electrolytic solution. According to an embodiment of the invention, it is possible to detect a status and/or a reaction product of the reaction by time-dependently measuring the electric conductivity of the electrolytic solution. Detectable reactions include: (a) an immune reaction, (b) an enzyme reaction, and (c) other chemical reactions including a binding reaction, a polymerization reaction, a decomposition reaction, and a catalytic reaction. Substances associated with the detectable reactions include: (i) proteins including a purified protein and a synthetic protein, (ii) enzymes, (iii) antigens including (i) and (ii) above, (iv) antibodies including polyclonal antibodies and monoclonal antibodies, and (v) other chemical substances. A specific substance in a sample can be detected and/or quantitatively determined by detecting a reaction between the specific substance and a substance reactive with the specific substance as the reaction. The specific substance may be a substance associated with a specific symptom of a disease, a product of a gene associated with a specific symptom of a disease, or an antibody against the same. According to an embodiment, the specific substance may be a cancer-related substance including a carcinoembryonic protein, a hormone, a hormone receptor, a membrane antigen, and a cancer-related gene product; or an antibody against these substances. The sample may be a body fluid including blood, serum, plasma, urine or ascites; a tissue or a tissue extract; or a cell or a cell extract.

In the first aspect of the invention, according to an embodiment, the method of the invention further comprises measuring a temperature of the electrolytic solution and conducting a temperature correction of a measured value of electric conductivity. According to another embodiment, the method of the invention further comprising adopting any one or a combination of: (1) maintaining an atmosphere outside the reaction system in which the reaction of the substance takes place in the electrolytic solution at a constant temperature; (2) thermally shielding the reaction system from the external atmosphere; and (3) maintaining the reaction system and the external atmosphere at the same temperature, without conducting a temperature correction of a measured value of electric conductivity. Furthermore, according to another embodiment, the method of the invention further comprising adopting any one or a combination of (1), (2) and (3) above, without conducting a temperature correction of a measured value of electric conductivity, and detecting the reaction status and/or the reaction product of the reaction of the substance in the electrolytic solution by time-dependently measuring a temperature of the electrolytic solution and measuring an amount of change or a rate of change in electric conductivity per unit temperature.

According to a second aspect of the invention, there is provided an immune reaction detecting method comprising detecting an immune reaction between an antigen and an antibody in an electrolytic solution on the basis of measurement of an electric conductivity of the electrolytic solution.

According to a third aspect of the invention, there is provided an immune reaction detecting method comprising detecting an immune reaction between an antigen and an antibody in a subject solution on the basis of measurement of a temperature of the subject solution.

According to a fourth aspect of the invention, there is provided a reaction detecting apparatus comprising: a reactor for containing a reactant and an electrolytic solution; electric conductivity detecting means issuing a signal corresponding to an electric conductivity of the electrolytic solution in the reactor; and control means detecting a signal issued by the electric conductivity detecting means in response to a reaction of a substance in the electrolytic solution. According to an embodiment of the invention, the reaction detecting apparatus further comprises temperature detecting means issuing a signal corresponding to a temperature of the electrolytic solution in the reactor to correct a measured value of electric conductivity based on an output of the electric conductivity detecting means on the basis of an output of the temperature detecting means. According to another embodiment of the invention, the reaction detecting apparatus further comprises any one or a combination of: (1) means for maintaining an atmosphere outside the reactor at a constant temperature; (2) means for thermally shielding the interior of the reactor from the atmosphere outside the reactor; and (3) means for maintaining the interior of the reactor and atmosphere outside the reactor at the same temperature. According to still another embodiment, the reaction detecting apparatus further comprises temperature detecting means issuing a signal corresponding to a temperature of the electrolytic solution in the reactor, wherein the control means generates a signal corresponding to an amount of change or a rate of change in electric conductivity per unit temperature on the basis of a signal issued by the electric conductivity detecting means in response to the reaction of the substance in the electrolytic solution, and a signal issued by the temperature detecting means in response to the reaction of the substance in the electrolytic solution.

According to a fifth aspect of the invention, there is provided an immune reaction detecting apparatus comprising: a reactor for containing a subject solution containing an antigen and an antibody; temperature detecting means issuing a signal corresponding to a temperature of the subject solution in the reactor; and control means detecting a signal issued by the temperature detecting means in response to an immune reaction between the antigen and the antibody in the subject solution.

According to a sixth aspect of the invention, there is provided an immune reaction detecting apparatus comprising: a reactor for containing a subject solution containing an antigen and an antibody; temperature detecting means issuing a signal corresponding to a temperature of the subject solution in the reactor; and control means detecting a signal issued by the temperature detecting means in response to an immune reaction between the antigen and the antibody in the subject solution.

Brief Description of the Drawings

Fig. 1 is a schematic view for explaining the principle of the reaction detecting method of the present invention: (A) illustrates ions moving in an electrolytic solution, and (B) illustrates ions moving when adding a substance reactive in the electrolytic solution;

Fig. 2 is a graph illustrating a time-dependent change in electric conductivity in a reaction between K1 antibody and a standard BFP (basic fetal protein) antigen in a physiological saline;

Fig. 3 is a graph illustrating a time-dependent change in electric conductivity in a reaction between K1 antibody and a carcinoembryonic antigen (CEA) in a physiological saline;

Fig. 4 is a graph illustrating time-dependent changes in electric conductivity of physiological salt solutions singly containing K1 antibody or three kinds of standard BFP antigens having different concentrations, respectively;

Fig. 5 is a graph illustrating a time-dependent change in electric conductivity in a reaction between K1 antibody and a standard BFP antigen in a physiological saline containing fetal calf serum (FCS) added thereto;

Fig. 6 is a graph illustrating the relationship between the amount of a standard BFP antigen and the amount of change in electric conductivity for each section of lapse of reaction time in a reaction between K1 antibody and a standard BFP antigen in a physiological saline containing fetal calf serum (FCS) added thereto;

Fig. 7 is a graph illustrating a time-dependent change in electric conductivity in a reaction between K1 antibody and a subject serum in a physiological saline;

Fig. 8 is a graph illustrating a time-dependent change in electric conductivity in a reaction between an MDM2 antigen and an MDM2 antibody in a physiological saline containing fetal calf serum (FCS) added thereto;

Fig. 9 is a graph illustrating the relationship between the amount of an MDM2 antibody and an amount of change in electric conductivity for each section of lapse of reaction time in a reaction between an MDM2 antigen and an MDM2 antibody in a physiological saline containing fetal calf serum (FCS) added thereto;

Fig. 10 is a graph illustrating a time-dependent change in electric conductivity in a reaction between an MDM2 antigen and a subject serum in a physiological saline;

Fig. 11 is a graph illustrating a time-dependent change in electric conductivity in a reaction between K1 antibody and pepsin in a physiological saline;

Fig. 12 is a graph illustrating the relationship between a change in temperature and a change in electric conductivity of a physiological saline;

Fig. 13 is a graph illustrating the amount of change in electric conductivity per 1°C of the solution temperature and a temperature coefficient of a physiological saline;

Fig. 14 is a graph illustrating time-dependent changes in electric conductivity and solution temperature, without conducting temperature correction, in a reaction between K1 antibody and a standard BFP antigen in a physiological saline;

Fig. 15 is a graph illustrating: (A) a time-dependent change in the amount of change in electric conductivity per 1°C; and (B) a time-dependent change in temperature coefficient, in a reaction between K1 antibody and a standard BFP antigen in a physiological saline;

Fig. 16 is a graph illustrating a time-dependent change in electric conductivity and in solution temperature, without temperature correction, of a physiological saline singly containing K1 antibody;

Fig. 17 is a graph illustrating a time-dependent change in electric conductivity and in solution temperature, without temperature correction, in a reaction between K1 antibody and a standard BFP antigen in a physiological saline containing fetal calf serum (FCS) added thereto;

Fig. 18 is a graph illustrating: (A) a time-dependent change in the amount of change in electric conductivity per 1°C; and (B) a time-dependent change in temperature coefficient, in a reaction between K1 antibody and a standard BFP antigen in a physiological saline containing fetal calf serum (FCS) added thereto;

Fig. 19 is a graph illustrating time-dependent changes in electric conductivity and solution temperature in a reaction between K1 antibody and subject serum ((A) serum of a healthy person (normal healthy subject); (B) serum of a cancer patient), without temperature correction, in a physiological saline containing fetal calf serum (FCS) added thereto;

Fig. 20 is a graph illustrating time-dependent changes in the amount of change in electric conductivity per 1°C and time-dependent changes in temperature coefficient, in a reaction between K1 antibody and subject serum ((A) serum of a healthy person; (B) serum of a cancer patient) in a physiological saline containing fetal calf serum (FCS) added thereto;

Fig. 21 is a graph illustrating a time-dependent change in electric conductivity in a reaction between K1 antibodies having different concentrations and subject serum ((A) serum of a healthy person; (B) serum of a cancer patient), without temperature correction, in a physiological saline containing fetal calf serum (FCS) added thereto;

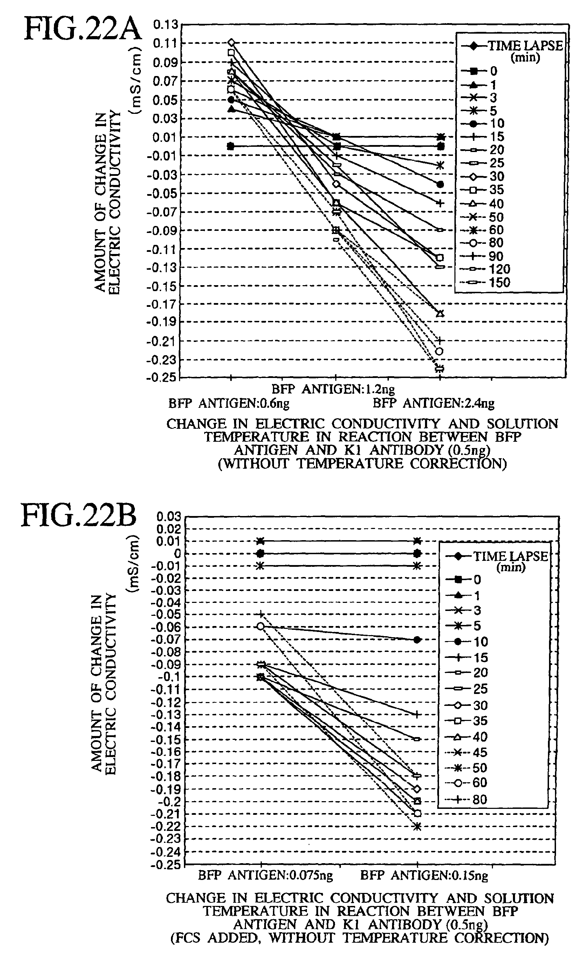

Fig. 22 is a graph illustrating: (A) the relationship between the amount of standard BFP antigen and the amount of change in electric conductivity for each division of lapse of reaction time, without temperature correction, in a reaction between K1 antibody and standard BFP antigen in a physiological saline; and (B) the relationship between the amount of standard BFP antigen and the amount of change in electric conductivity for each division of lapse of reaction time, without temperature correction, in a reaction between K1 antibody and standard BFP antigen in a physiological saline containing fetal calf serum (FCS) added thereto;

Fig. 23 is a schematic view illustrating an outline of configuration of an embodiment of the reaction detecting apparatus of the invention;

Fig. 24 is a schematic view illustrating an outline of configuration of another embodiment of the reaction detecting apparatus of the invention; and

Fig. 25 is a schematic view illustrating an outline of configuration of an embodiment of the immune reaction detecting apparatus of the invention.

Best Mode for Carrying Out the Invention

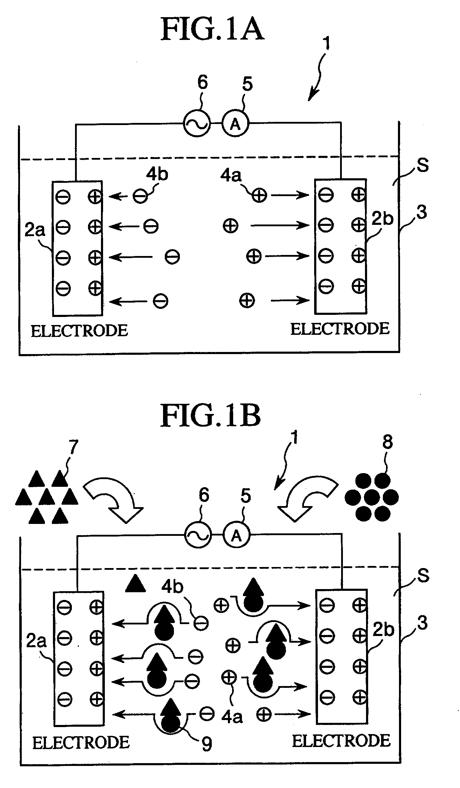

The principle of the present invention will first be described with reference to Fig. 1. As shown in Fig. 1A, in an electrolytic solution S contained in a container 3, electrolyte is dissociated into cations 4a and anions 4b. When detecting electric conductivity of this electrolytic solution S, a pair of electric conductivity measuring electrodes of an electric conductivity meter 1 (electric conductivity measuring cell, hereinafter simply referred to as "cell") 2 (2a and 2b) are immersed in the electrolytic solution S, and an electric conductivity measuring power source (AC power source) 6 electrically connected to these electrode pair 2a and 2b is turned on. This charges a + (positive) pole and a - (negative) pole on the surface of the electrode pair 2a and 2b. Anions 4b of electrolyte move to the + (positive) pole, and cations 4a of electrolyte move to the - (negative) pole on the electrode pair 2a and 2b, and electric current flows as a result. This current is measured with an ammeter 5 connected to the electrode pair 2a and 2b to calculate electric conductivity of the electrolytic solution S. In this state, electric conductivity of the electrolytic solution S depends upon electrolyte concentration in the solution.

On the other hand, addition of a substance reactive in the electrolytic solution (reactant) to the electrolytic solution S is considered. As shown in Fig. 1B, when a reaction product (complex) 9 is generated by adding, for example, two kinds of substance 7 and 8, and through binding of these two substances, electric conductivity of the electrolytic solution S varies with generation of the reaction product 9, and electric conductivity becomes lower in this case. Not intending to be bound by a particular theory alone, studies carried out by the present inventors suggest that generation of the reaction product (complex) 9 through binding of the two substances 7 and 8 inhibits movement of ions (cations and anions) 4a and 4b. As a result, flow of current becomes difficult (resistance becomes larger), leading to a lower electric conductivity.

When, through reaction of substances in the electrolytic solution, for example, reaction of two or more substances causes decomposition of any of the substances, or a substance becomes smaller substances through decomposition, movement of ions becomes easier (resistance becomes lower), and electric conductivity becomes higher, contrary to the above.

The International Publication No. WO96/30749 discloses a method for determining the concentration of a nonelectrolyte present in an electrolytic solution on the basis of measurement of electric conductivity. For example, it is demonstrated that sequential addition of glucose which is a nonelectrolyte to an electrolytic solution mainly containing sodium chloride as an electrolyte causes a change (decrease) in electric conductivity of the electrolytic solution, and as a result, the amount of nonelectrolyte to be added to the electrolytic solution, i.e., concentration is determined, by use of the correlation between the concentration of nonelectrolyte and electric conductivity of the electrolytic solution previously determined within the same system.

In this known technique, however, the nonelectrolyte of which concentration is to be measured is existent singly in the electrolytic solution, and is not accompanied by a reaction or an interaction in the electrolyte. That is, the known technique is not the one to detect a reaction of substances in the electrolytic solution.

As described above, the present inventors found the possibility to detect a reaction of substances in the electrolytic solution by measuring electric conductivity of the electrolytic solution, on the basis of the novel findings that the manner of ion movement in the electrolytic solution varied in response to the status of reaction and generated reaction products of the reaction of substances in the electrolytic solution.

By detecting a change in electric conductivity of the electrolytic solution caused along with a reaction of substances in the electrolytic solution, it is possible to detect reaction products generated by the reaction of substances, such as a complex produced by binding of two substances, and decomposition products generated by the reaction of substances.

By time-dependently measuring a change in electric conductivity of the electrolytic solution, it is possible to time-dependently detect the status of reaction of substances in the electrolytic solution, and hence to easily and electrically grasp the status of progress of a binding reaction or a decomposition reaction. This is useful for analyzing the time-dependent status of reaction in a reaction of substances, and in addition, the possibility to observe a reaction in a real-time manner permits, for example, easy selection of an antibody easily reactive with an antigen in an immune reaction, or easily and electrically determine presence of sensitivity between an antigen and an antibody.

According to the present invention, it is possible to detect a specific substance in a sample, i.e., to confirm whether or not a specific substance is present in a sample. In order to detect a specific substance existing in the sample, it suffices to use a substance specifically reactive with the specific substance, and detect the reaction between these substances (status of reaction and reaction products).

According to the invention, a specific substance in a sample can be quantitatively determined. Dependency of a measured result of electric conductivity of the electrolytic solution on the quantity (concentration) of the specific substance is previously determined in the same system, regarding reaction of the specific substance of which concentration is to be measured and a substance specifically reactive with this specific substance. For example, concentration of the substance specifically reactive with the specific substance of which concentration is to be measured is kept constant. Dependency of the measured result of electric conductivity on concentration of the specific substance in reaction between the specific substance and the substance specifically reactive therewith is then previously determined as a calibration curve, relative to various values of concentration of the specific substance of which concentration is to be measured.

The calibration curve may be appropriately prepared in response to the feature of each reaction. For example, the relationship between the amount of change in electric conductivity and concentration of the specific substance upon lapse of a prescribed time period after start of reaction, the relationship between the reaction rate (time-dependent rate of change of electric conductivity such as an initial rate of reaction) and concentration of the specific substance, and the relationship between the value of electric conductivity and concentration of the specific substance at the time when the change in electric conductivity is saturated can be previously determined as a calibration curve. Those skilled in the art can select a calibration curve the most suitable for a target reaction. Or, it is possible to evaluate the quantity (concentration) of the specific substance in the electrolytic solution by comparing a measured value of electric conductivity to a prescribed threshold value (cutoff value). This threshold value suffices, like the calibration curve described above, to be appropriately set in response to the feature of each reaction, for example, as the above-mentioned reaction rate (time-dependent rate of change of electric conductivity, such as the initial reaction rate). The term "quantitative determination" as used here includes evaluation or comparison of the quantity of the specific substance, and a more detailed quantitative determination.

In the invention, there is essentially no restriction imposed on the reaction of substances to be detected. The reactive substance (reactant) may be any substance(s) so far as being reactive in the electrolytic solution, irrespective of the kind and the number thereof. For example, it is possible to detect a decomposition reaction of a substance in the electrolytic solution, which is decomposed into at least two substances, or a binding reaction between at least two substances reacting in the electrolytic solution. Reactive substances may naturally be more. As is clear from the aforementioned principle, the reaction must take place in the electrolytic solution having a concentration at which electric conductivity can be measured at a desired accuracy.

For example, a reaction associated with biological molecules such as an immunological reaction or an enzymatic reaction is a typical subject of detection in the invention. Applicable reactant include a purified protein, a synthetic protein, an enzyme, an antigen and an antibody. Among others, an immune reaction between an antigen and an antibody is the most typical object of detection of the invention.

Any reaction which can take place in the electrolytic solution such as a polymerization reaction, a binding reaction, a decomposition reaction, a catalytic reaction (catalysis) or any other chemical reaction can be detected. In other words, it is possible to detect a reaction in which reaction between substances can generate a larger substance (a polymerization reaction or a binding reaction), a reaction in which substances can be decomposed by light, ultraviolet rays or temperature to generate smaller substances (a decomposition reaction), or a reaction in which any of the substances in reaction between at least two substances can be decomposed to generate smaller substances (a decomposition reaction), and furthermore, a reaction in which addition of a substance bringing about a more remarkable effect of the above-mentioned reactions causes an increase in reaction rate (a catalytic reaction). By detecting these reactions, it is also possible to detect and quantitatively determine a specific substance in a sample.

According to the invention, in particular, it is possible to detect a reaction between a specific substance associated with a specific state of a disease and a substance reactive therewith, and detect and quantitatively determine the specific substance existent in a sample. More specifically, the specific substance associated with the specific state of a disease is a substance which specifically expresses or excessively express relative to a specific state of a disease in vivo, a specific gene product such as a partial peptide of a specific gene to a specific disease, or an antibody produced against these substances.

A specific state of a disease is, for example, cancer, and the specific substances to cancer, i.e., the cancer-related substances include: tumor markers (tumor-related antigens) represented by carcinoembryonic proteins such as $\alpha$-fetoprotein (AFP), basic fetal protein (BFP), and carcinoembryonic antigen (CEA); various hormones known to excessively express in hormone-dependent cancer tissue and receptors thereof; and other gene products considered to be deeply associated with cancer.

According to the invention, it is possible to simply detect and quantitatively determine these cancer-related substances or antibodies produced in vivo against these substances.

For example, these substances include a blood autoantibody to cancer gene (oncogene) product MDM2 (Murine Double Minute 2) playing an important role in carcinogenesis and progress of cancer as a decomposition enzyme of cancer suppressor gene p53 protein, and autoantibodies in blood against hormones of which excessive expression in a hormone-dependent cancer tissue is observed at a high frequency and receptors thereof (estrogen receptor (ER), androgen receptor (AR)).

These cancer-related substances and cancer-related gene products do not easily move outside the cell nucleus since they are factors within cell nucleus. In the initial stage of carcinogenesis, however, cancer cells are attacked and broken by host immune reaction. As a result, factors in nucleus move to outside cells, and humoral antibodies (for example, MDM2 protein autoantibody, ER protein autoantibody, AR protein autoantibody) are predicted to occur in the cancer host blood. Detection of these autoantibodies in blood is therefore expected to be useful for early diagnosis of cancer, since the autoantibodies are consider to occur in an early stage of carcinogenesis.

There is no particular restriction imposed on the sample to which detection and quantitative determination of a specific substance is applicable. As described above, when detecting and quantitatively determining a specific substance associated with a specific state of a disease, the sample is typically a body fluid sampled from a mammalian for analysis including blood, serum, plasma, urine and ascites; a tissue or an extract thereof; or a cell or an extract thereof. More preferably, the sample is a human body fluid sampled for analysis including human blood, serum, plasma, urine and ascites; an extract of human tissue; or extract of human cell. This permits detection and quantitative determination of a specific substance in body fluid of a human patient, particularly, a substance associated with a specific state of a disease. This is useful for diagnosing as to whether or not the human patientsuffers from the specific disease, or the state of the disease. By using a specific tissue or an extract thereof, or a cell or an extract thereof as a sample, it is possible to easily measure an organ specific reactivity of a specific reaction to be detected, thus providing a remarkable advantage.

When detecting an antigen suspected to be present in a sample, it suffices to cause reaction between the sample and an antibody specifically reactive with this antigen in an electrolytic solution, and detecting the status of reaction of these substances and reaction products (immune complex) on the basis of measurement of electric conductivity. In order to detect an antibody in the sample, on the other hand, it suffices to use an antigen specifically reactive with this antibody, and conduct detection of the status of reaction of these substances and reaction products (immune complex) on the basis of measurement of electric conductivity. The antigen used for reaction may be a substance which can be an antigen, such as a purified antigen, a chemically synthesized antigen or a genetic recombination antigen. The antibody used for the reaction may be a commercially available antibody, or a purified antibody specifically prepared to an antigen. This antibody may further be a polyclonal antibody or a monoclonal antibody.

In the invention, no particular restriction is imposed on the electrolytic solution, i.e., on the kind of electrolytes and the number thereof. The electrolytic solution is however selected in view of the reaction taking place therein. That is, from the point of view of measuring electric conductivity of the electrolytic solution, a change in ion transfer can be recognized more easily when ion dissociation constant of electrolyte is higher. It is also necessary to maintain stability of reactants in the electrolytic solution. It is important to select an electrolyte and concentration thereof, taking account of these requirements.

An aqueous solution containing sodium ions, potassium ions and calcium ions is suitably applicable as an electrolytic solution.

For example, when detecting an immunological reaction, a physiological saline (an aqueous NaCl solution of 0.15 M), or a potassium chloride solution (for example, an aqueous KCl solution of 0.15 M) is suitably applicable. In this case, the electrolytic solution should preferably have a pH within a range of from 6.0 to 8.0, or more preferably, about 7.0.

When the most suitable pH is in the acidic region or in the alkaline region in an enzymatic reaction or any of the other chemical reactions (polymerization reaction, binding reaction, decomposition reaction, and catalytic reaction) as described above, hydrochloric acid (aqueous HCl solution) or sodium hydroxide (aqueous NaOH solution), not limitative, can be used as an electrolytic solution or a pH adjusting reagent.

In the invention, the electric conductivity meter used for measuring electric conductivity may be a commercially available meter without a particular limitation. For cell as well, a commercially available one may be used without any particular restriction. An electric conductivity meter having a desired performance should naturally be used in order to obtain a desired detection accuracy.

Examples of the present invention will now be described further in detail with reference to concrete results of measurement.

Example 1

In this example, the time-dependent status of reaction and an immune complex will be detected on the basis of measurement of electric conductivity for an immune reaction between a purified antigen and an antibody specifically reactive with the purified antigen.

A purified specimen of BFP (basic fetal protein), which was known as a tumor marker and generally used in the art, having a molecular weight of about 55,000 (hereinafter referred to as "BFP antigen") was used as the antigen. The purified specimen of BFP antigen (hereinafter referred to as "standard BFP antigen") was autopurified from nude mouse-transplanted human hepatoma cells. The standard BFP antigen was purified as follows in accordance with a common practice generally known to those skilled in the art. Homogenate of nude mouse-transplanted human hepatoma cells were subjected to affinity chromatography using a polyclonal BFP rabbit antibody, then to gel filtration column chromatography. The protein concentration was quantitatively determined by the Lowry-Folin method. The BFP antigen activity per protein concentration was confirmed by the EIA method and the Ouchterlony method. The result showed a purification purity of the standard BFP antigen of 99.99%. In all the following examples, identical standard BFP antigens were used.

A mouse monoclonal antibody homemade by the cell fusion method with a standard BFP antigen purified from human hepatoma cells as an immunogen (hereinafter referred to as "K1 antibody") (molecular weight: about 150,000) was used as the antibody. The K1 antibody was prepared as follows in accordance with the common procedure well-known to those skilled in the art. Hybrid cells were cloned by the limiting dilution method. The cloned hybrid cells were inoculated into the abdominal cavity in a number of $10^6$ cells per BALB/c mouse, and after the lapse of ten days, the BFP antibody was sampled as ascites. After ammonium sulfate fractionation of the ascites, the antibody was obtained through purification by ion exchange chromatography with DEAE cellulose. In all the following examples, the same K1 antibody was used.

A physiological saline (aqueous NaCl solution of 0.15 M) was used as the electrolytic solution. Unless otherwise specified, the electrolytic solution had a pH of 7, and the solution had room temperature (about 26°C) at the start of reaction. Because an excessively low electrolyte concentration in the solution makes it impossible to measure electric conductivity itself, the electrolyte concentration must be high to such extent as to permit measurement of electric conductivity. The physiological saline (0.15 M) used in this example posed no problem in implementing the method of the invention. In all the following examples, the same electrolytic solution was used.

A CM30V digital electric conductivity meter made by TOA Electronics Ltd. (DKK TOA Corporation at present) (hereinafter simply referred to as "electric conductivity meter") was used as the electric conductivity meter. The electric conductivity meter has a cell (electrode pair for measuring electric conductivity) and calculates electric conductivity, by impressing an AC voltage (peak to peak voltage) Vp-p = about 100 mV to the cell and measuring the amount of current flowing between electrodes.

Electric conductivity varies with the solution temperature. The electric conductivity meter used in this example had an automatic temperature compensation (ATC) function which detects solution temperature by use of thermistor, sets a solution temperature coefficient, and automatically correct changes in electric conductivity caused by a change in solution temperature. A thermistor is built in the cell, which had an accuracy of 1/10°C. In all the following examples, the same electric conductivity meter was used.

In the following examples, furthermore, the same reactor and other measuring instruments as in this example are used in common.

Measuring procedure

An amount of 10 ml of physiological saline was provided in a small-capacity vial (capacity: 12 ml), and the cell of an electric conductivity meter was immersed in this vial. Then, an amount of 2 µl (absolute amount:

0.5 ng) of K1 antibody adjusted with a physiological saline to a concentration of 0.25 µg/ml was added with a microsyringe into this vial and stirred. Subsequently, an amount of 2 µl (absolute amount: 1.2 ng) of standard BFP antigen adjusted with a physiological salt solution to a concentration of 0.6 µg/ml was added into this vial by use of a microsyringe. After stirring this reaction solution, electric conductivity was time-dependently measured while keeping the cell as immersed in the reaction solution.

Similarly, two batches of standard BFP antigen adjusted to concentrations of 0.3 µ g/ml and 0.15 µg/ml with physiological saline (absolute amounts: 0.6 ng and 0.3 ng) were added by means of a microsyringe to a physiological saline containing K1 antibody (absolute amount: 0.5 ng) and stirred as described above, and then, electric conductivity was time-dependently measured.

A carcinoembryonic antigen (CEA) (available from International Enzymes Company) (absolute amount: 0.5 ng) was added, in place of the standard BFP antigen, to a physiological saline containing K1 antibody (absolute amount: 0.5 ng), and then, electric conductivity was time-dependently measured.

Furthermore, K1 antibody (absolute amount: 0.5 ng) and standard BFP antigen (absolute amounts: 0.3 ng, 0.6 ng and 1.2 ng) were independently added to a physiological saline (0.15 M), respectively, and electric conductivity was time-dependently measured.

Result

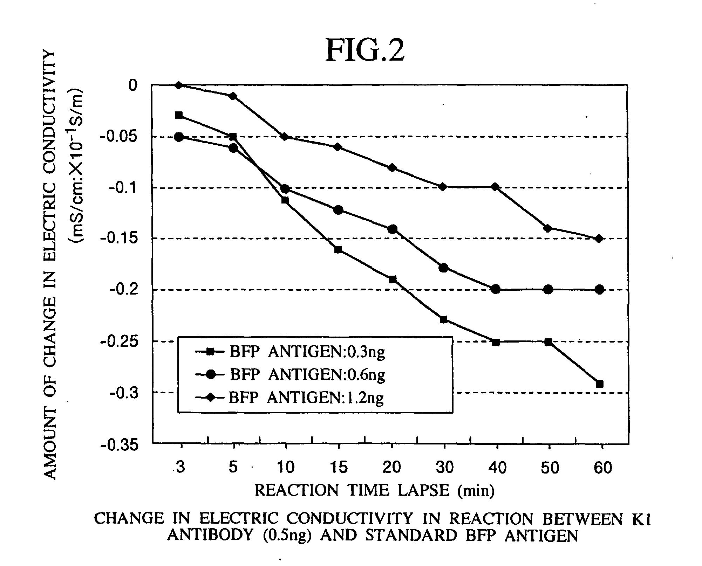

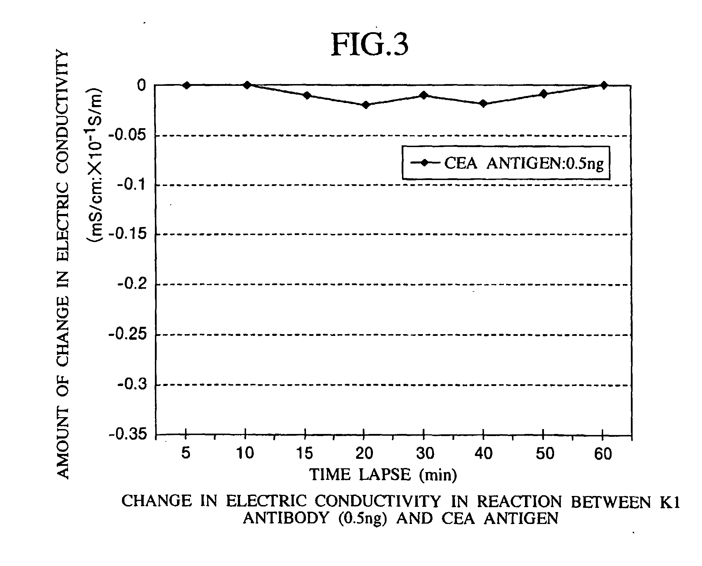

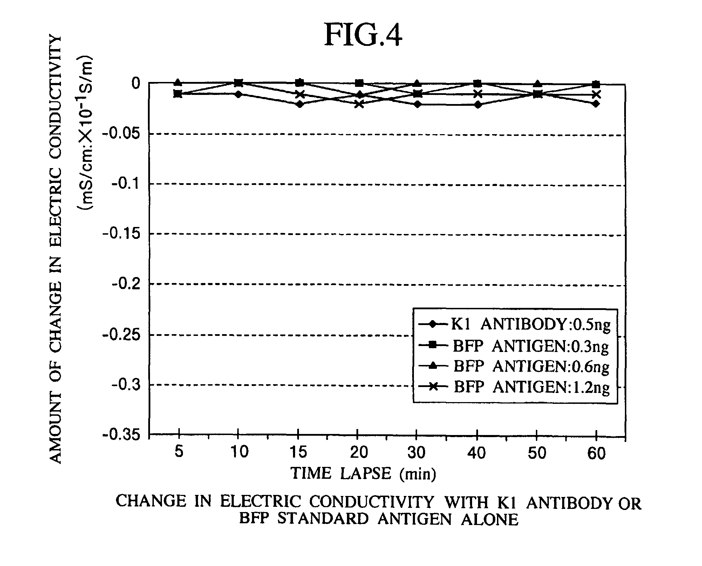

In reactions of K1 antibody (0.5 ng) with standard BFP antigens having respective concentrations (1.2 ng, 0.6 ng and 0.3 ng), measured values of electric conductivity at points in time lapse are shown in Fig. 2. Values of electric conductivity at points in time lapse of a physiological saline containing K1 antibody (0.5 ng) and carcinoembryonic antigen (CEA) (0.5 ng) are shown in Fig. 3. Furthermore, values of electric conductivity at points in time lapse of physiological saline singly containing K1 antibody (0.5 ng) and BFP antigens (0.3 ng, 0.6 ng and .12 ng) are shown in Fig. 4.

In Figs. 2 to 4, measured values of electric conductivity are represented by amounts of change in electric conductivity obtained by using the electric conductivity a minute after the start of reaction as a blank value, and subtracting the blank value from values of electric conductivity at points in time lapse.

As seen in Fig. 2, in the reaction between K1 antibody and standard BFP antigen, electric conductivity changes to smaller values along with the lapse of reaction time.

It is understood that a formation of immune complex through immune reaction between antigen and antibody prevents movement of ions, i.e., $Na^+$ and $Cl^-$ of the physiological saline in this example, in the electrolytic solution, and this causes a decrease in electric conductivity. According to the invention, as described above, it is possible to know a change in the manner of ion movement in the electrolytic solution caused by reaction products and detect reaction products specific to such a reaction.

Under the reaction conditions in this example, electric conductivity changed to a lower value at a lower concentration (0.3 ng) than at a higher concentration (1.2 ng) of the standard BFP antigen. Not intending to be bound by a particular theory, these results suggest quantitative adaptability of each substance in the immune reaction between antigen and antibody, reflecting the fact that an amount of standard BFP antigen of 0.3 ng tends to more easily cause a reaction than an amount of 1.2 ng when the amount of K1 antibody is kept constant (0.5 ng).

By measuring electric conductivity of the reaction solution, as described above, it is possible to time-dependently detect the status of reaction of the immune reaction specific to standard BFP antigen and K1 antibody in the electrolytic solution, as electric conductivity. It is also possible to easily measure reaction properties in the electrolytic solution such as the dependency of the reaction between K1 antibody and standard BFP antigen on concentration of standard BFP antigen.

Further, as is evident from reference to the results shown in Figs. 3 and 4, the result of measurement in this example reveals that:

(1) The status of reaction (reaction properties) in which the immune reaction between standard BFP antigen and K1 antibody depends upon concentration of the standard BFP antigen with a constant concentration of K1 antibody can be grasped as changes of electric conductivity;
(2) Electric conductivity tends to show a lower value with the lapse of time;
(3) Addition of an antigen other than a BFP antigen which is known to be non-reactive with K1 antibody, i.e., a carcinoembryonic antigen (CEA) in this example in place of the standard BFP antigen does not lead to time-dependent decrease in electric conductivity, and dependency of a change in electric conductivity upon antigen concentration is not observed (Fig. 3); and
(4) In addition, even in the individual presence of K1 antibody and the standard BFP antigen in a similar electrolytic solution, electric conductivity does not show a time-dependently decreasing tendency (Fig. 4).

Example 2

Another example in which an immune reaction in an electrolytic solution was detected will now be described.

Measuring procedure

In this example, an amount of 2 µl (about 140 µg protein in absolute amount) of fetal calf serum (FCS) containing about 70 mg protein/ml FCS was previously added by use of a microsyringe to 10 ml physiological saline, and changes in electric conductivity in each reaction between K1 antibody (0.5 ng) and standard BFP antigens of different concentrations (0.6 ng, 1.2 ng and 2.4 ng) were time-dependently measured in the same measuring procedure as in Example 1.

Fetal calf serum was used for achieving an amount of protein corresponding to the amount of addition to the reaction system when evaluating and quantitatively determining the amount of BFP antigen contained in a subject serum in the examples described later. It is known that the fetal calf serum is not reactive with the K1 antibody.

Result

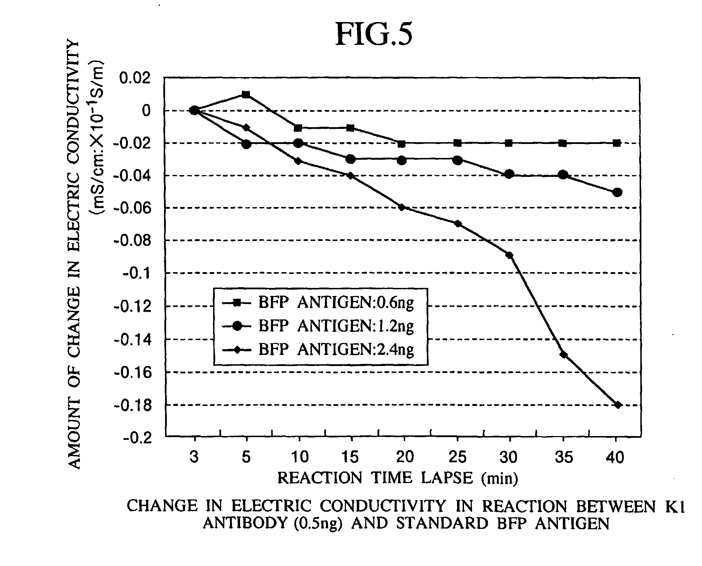

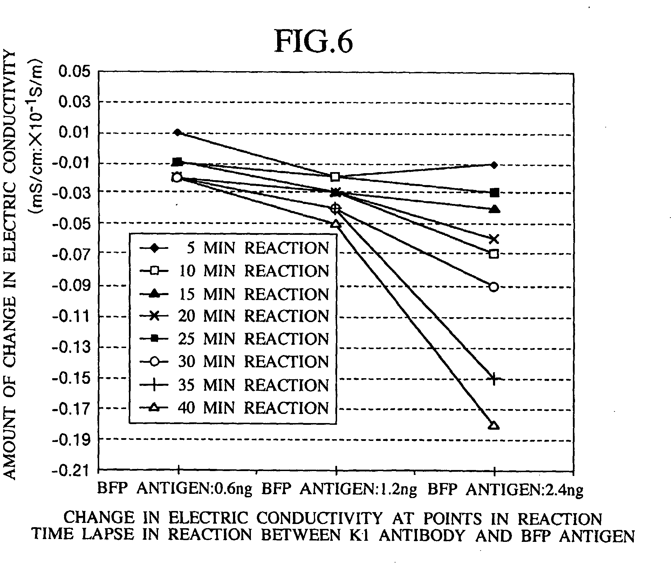

The result is shown in Fig. 5. In Fig. 5, measured values of electric conductivity are represented by amounts of change in electric conductivity obtained by using the electric conductivity a minute after the start of reaction as a blank value, and subtracting the blank value from values of electric conductivity at points in time lapse. Amounts of change in electric conductivity for individual amounts of standard BFP antigen during divisions of reaction time are shown in Fig. 6.

Figs. 5 and 6 reveal that, for each reaction, electric conductivity decreases with the lapse of reaction time.

As is clear from Fig. 5, when adding fetal calf serum to the reaction system, the amount of change (decrease) in electric conductivity becomes larger according as the amount (concentration) of the standard BFP antigen is larger. This is considered attributable to the reflection of the status of reaction between BFP antigen and K1 antibody in resistance of serum protein as a result of addition of the fetal calf serum to the reaction system. This suggests that, in this state, a larger amount of BFP antigen leads to a better reactivity thereof relative to K1 antibody.

As is evident from the description in Examples 1 and 2, according to the invention, it is possible to very easily detect a reaction of substances in an electrolytic solution, without the need of an expensive and large-scaled equipment. Also, according to the invention, it is possible to detect not only a finally formed immune complex (as in the conventional immunological measuring methods (RIA and EIA methods)), but also the time-dependent status of reaction of substances and reaction products in the electrolytic solution.

As is clear from the above, an immune reaction between an antigen and an antibody can be time-dependently measured. It is therefore possible to very easily, rapidly and in a real-time manner accomplish, for example, selection of an antibody more easily reactive with an antigen or an antigen more easily reactive with an antibody, and measurement for determining presence of sensitivity between an antigen and an antibody.

According to the invention, furthermore, an immune reaction can be detected rapidly and simply by adding a reactant directly to the electrolytic solution without the need for such operations as solidification of an antigen or an antibody into a carrier and preparation of a labelled substance as in the conventional immunological measuring methods (RIA and EIA methods). This permits detection of an immune reaction by use of a very simple apparatus without generating waste of reagents such as labelled substances and coloring agents (enzyme substrates) or solidification carriers.

Example 3

In this example, the presence of a BFP antigen in serum is detected as a specific substance in a sample by use of an anti-BFP mouse monoclonal antibody (K1 antibody) which forms an immune complex through specific reaction with the BFP antigen.

In this example, furthermore, the amount of BFP antigen in serum is evaluated and quantitatively determined by use of a K1 antibody. The BFP antigen contained in serum reacts with K1 antibody in accordance with the amount thereof. It is therefore possible to evaluate and quantitatively determine BFP antigen contained in serum through time-dependent measurement of electric conductivity of the reaction solution, by previously determining the status of reaction (reaction properties) between BFP antigen and K1 antibody dependent upon the amount (concentration) of BFP antigen in the same system.

In this example, human sera of a healthy person (normal healthy subject) and a cancer patient (hepatoma patient) freeze-stored at -20°C for test use. The human sera of the healthy person and the cancer patient used in this example contained BFP antigen in amounts of 24.8 ng/ml and 540 ng/ml, respectively, as measured by a standard EIA method using "Lanazyme (trade mark) BFP Plate" made by Nippon Kayaku Co., Ltd.

"Lanazyme (trade mark) BFP Plate" is based on the EIA method using two different kinds of mouse monoclonal BFP antibody (K1 antibody and 5C2 antibody) and sandwiching BFP antigen between K1 antibody solidified on a plate and horseradish-peroxidase labelled 5C2 antibody. This method comprises the steps of coloring the BFP antigen sandwiched between the two antibodies by use of 3,3',5,5'-tetramethylbenzidine (TMB) with urea hydrogen peroxide as a substrate, measuring absorbance of a wavelength of 405 nm, and quantitatively measure BFP antigen in the subject serum from the calibration curve prepared with standard BFP antigen. An antigen purified from nude mouse-transplanted hepatoma was used as the BFP antigen.

Measuring procedure

Measurement was performed in the same manner as in Examples 1 and 2. That is, an amount of 2 µl (absolute amount: 0.5 ng) of K1 antibody was added by means of a microsyringe to 10 ml physiological saline and stirred. An amount of 2 µl (about 140 µg protein in absolute amount) of subject serum (about 70 mg protein/ml) brought back to room temperature was added by use of a microsyringe to this solution and stirred. Electric conductivity was time-dependently measured while immersing the cell in this reaction solution.

Result

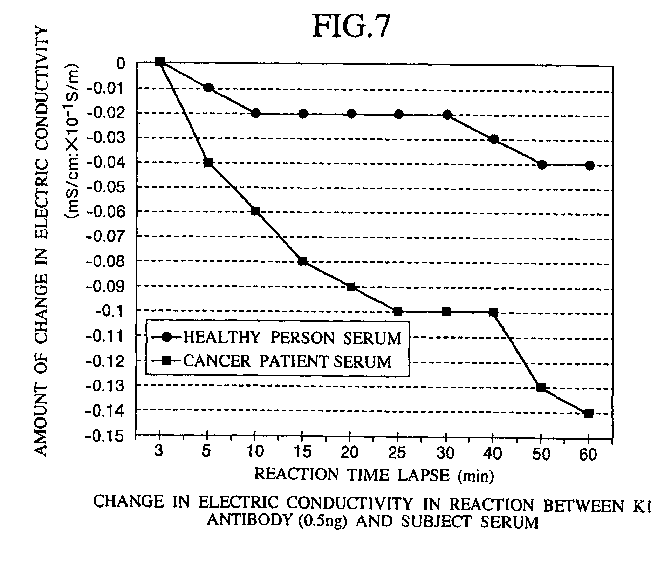

Measured values of electric conductivity at points in time lapse when adding subject sera (a healthy person and a cancer patient) to a physiological saline containing K1 antibody are illustrated in Fig. 7. In Fig. 7, measured values of electric conductivity are represented by amounts of change in electric conductivity obtained by using the electric conductivity a minute after the start of reaction as a blank value, and subtracting the blank value from values of electric conductivity at points in time lapse. The resultant values are shown as amounts of change in electric conductivity.

As shown in Fig. 7, by adding subject sera of the healthy person and the cancer patient, electric conductivity of the physiological saline containing K1 antibody decreased along with the lapse of time.

By time-dependently measuring electric conductivity of the electrolytic solution (physiological saline) which varies with formation of an immune complex by use of K1 antibody reactive specifically with the BFP antigen, as described above, it is possible to very easily detect the BFP antigen in samples (subject sera of the healthy person and the cancer patient), i.e., to confirm existence thereof.

As is clear from the result shown in Fig.7, there is a apparent difference between the subject serum of the healthy person and the subject serum of the cancer patient. More specifically, reactivity per time lapse in the reaction between the subject serum of the cancer patient and the K1 antibody is higher than that in the reaction between the subject serum of the healthy person and the K1 antibody.

In general, BFP antigen is present also in body fluid of a healthy person, and is known to show a higher value in body fluid of a cancer patient than in that of a healthy person. Positivity rates for a healthy person and a cancer patient at a prescribed cutoff value of BFP antigen are known: the positivity rate corresponding to a cutoff value of 75 ng/ml (EIA method) is 5% for healthy persons and 60 to 80% for cancer patients.

Time-dependent changes in electric conductivity of the reaction solution caused along with the reaction (reference reaction) between standard BFP antigen and K1 antibody of prescribed concentrations in serum, i.e., in amounts corresponding, for example, to the above-mentioned cutoff value of 75 ng/ml are previously determined in the same system. Thus, by measuring time-dependent changes in electric conductivity of the reaction solution caused along with the reaction with K1 antibody for a subject serum, and comparing reactivity per time lapse to reactivity of the previously determined reference reaction, it is possible to evaluate easily and in a real-time manner whether or not the BFP antigen is present in the sample in an amount of over the prescribed value.

In this example, as described above, BFP antigen is contained in the sera of the healthy person and the cancer patient in amounts of 24.8 ng/ml and 540 ng/ml, respectively, as determined by the EIA method. In other words, BFP antigen is present in an amount of about 50 pg in the healthy person serum and about 1 ng in the cancer patient serum in 2 μl of subject serum added to the reaction system, respectively.

Therefore, since an evident difference is observed in reactivity relative to K1 antibody between the healthy person serum and the cancer patient serum as described above, the possibility is understood to very easily detect the trace BFP antigen in an amount of about 50 pg to 1 ng through time-dependently measuring electric conductivity of the reaction solution. Further, if there is present a BFP antigen of at least 50 pg to I ng, it is very easy to time-dependently detect the behavior of immune reaction between BFP antigen and K1 antibody, and the amount of BFP antigen in the sample can be evaluated by very simply, rapidly and in a real-time manner measuring the difference in reactivity between the sample containing the BFP antigen and the K1 antibody.

Furthermore, the BFP antigen in the subject serum was quantitatively determined more in detail. In this example, a calibration curve (standard curve) used for quantitative determination of the BFP antigen in the subject serum was derived from the result of Example 2, in which fetal calf serum (FCS) as protein corresponding to the subject serum added to the reaction system was previously added (about 140 μg protein in absolute amount) to a physiological saline, and K1 antibody and the BFP antigen were caused to react.

For the preparation of the calibration curve, it suffices to select the most suitable calibration curve by use of the reaction between an antigen and an antibody having known concentrations in accordance with selection of reactivity between antigen and antibody, reaction conditions and a measuring range.

By plotting the result shown in Fig. 5 with the standard BFP antigen concentration on the abscissa, and changes in electric conductivity (negative values) on the ordinate, the relationship between the amount of standard BFP antigen and the amount of change in electric conductivity is obtained as shown in Fig. 6. In this example, this relationship tends to show linearity with the lapse of reaction time: for a lapse of reaction time of over 30 minutes, reactivity of high-concentration antigen tended to lead to a higher reactivity.

For example, in the relationship shown in Fig. 6, by using the relationship between the standard BFP antigen concentration and electric conductivity at the lapse of 40 minutes of reaction having linearity as a calibration curve, the amount of BFP antigen in the cancer patient serum measured by present method was calculated to be about 700 ng/ml (which is a value close to 540 ng/ml obtained by application of the EIA method). On the other hand, for the healthy person serum, since the BFP antigen is present in an amount of only 1/20 that in the cancer patient serum, the result was considered to reflect the state in which the amount of K1 antibody was excessive relative to the amount of BFP antigen in the serum of healthy person. The degree of agreement with the value obtained by the EIA method was lower than in the case of the cancer patient serum. For measurement of BFP in serum at a lower concentration, it suffices to adopt a smaller ratio of antigen to antibody, and select a more suitable calibration curve.

There is an evident difference in reactivity with K1 antibody between the healthy person serum and the cancer patient serum, showing an obvious difference in reactivity per lapse of time. It is therefore considered possible to quantitatively determine the amount of BFP antigen in a sample by comparing values of reactivity per lapse of time between the reaction of standard antigen of known concentrations with K1 antibody to the reaction between the subject serum and K1 antibody, this being commonly known as the rate-assay.

Example 4

As another example of detection of a specific substance in a sample, an blood autoantibody against MDM2 protein known as a cancer-related gene product was detected.

First, changes in electric conductivity accompanying reactions between MDM2 antigen and purified MDM2 antibodies in different amounts (concentrations) in a physiological saline were measured. As the MDM2 antigen, a synthetic MDM2 peptide antigen of 20-mer on the N-terminal side (available from Asahi Techno-Glass Co.) (molecular weight: about 1,991) was used. As the purified MDM2 antibody, a polyclonal rabbit antibody (available from Santa Cruz Biotechnology, Inc., hereinafter referred to as "standard MDM2 antibody") (molecular weight: about 150,000) against above-mentioned synthetic peptide antigen was used.

Then, MDM2 autoantibody in sera of a healthy person, a stomach cancer patient and a colon cancer patient freeze-stored at -20°C was detected. The same MDM2 antigen as above was used.

Measuring procedure

The measuring procedure was similar to that as in Examples 1 to 3. That is, MDM2 antigen (absolute amount: 200 ng) is first added to 10 ml physiological saline and stirred. Then, after adding standard MDM2 antibody (absolute amounts: 6.25 ng, 12.5 ng and 25.0 ng) to this solution and stirring the same, electric conductivity was time-dependently measured while keeping the cell immersed in the reaction solution.

For the purpose of ensuring adaptability to the reaction conditions upon detecting MDM2 autoantibody in the subject serum, i.e., to the amount of protein in the reaction system as in Example 2, an amount of 2 µl (absolute amount: about 140 µg) of fetal calf serum (FCS) (about 70 mg protein/ml) was added to the reaction system between the MDM2 antigen and the standard MDM2 antibody.

On the other hand, an amount of 2 µl (absolute amount: about 140 µg protein) of subject sera (about 70 mg protein/ml) of a healthy person, a stomach cancer patient and a colon cancer patient brought back to room temperature were added by means of a microsyringe into 10 ml physiological saline containing MDM2 antigen (absolute amount: 200 ng), respectively and stirred. Then, electric conductivity was time-dependently measured while keeping the cell immersed in the reaction solution.

Result

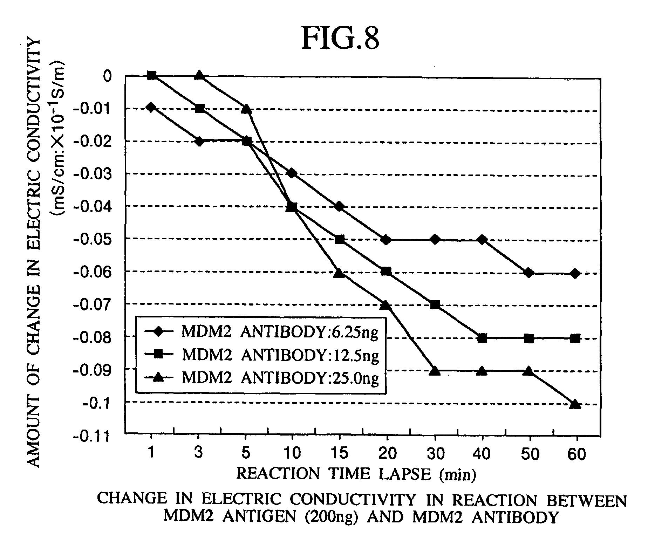

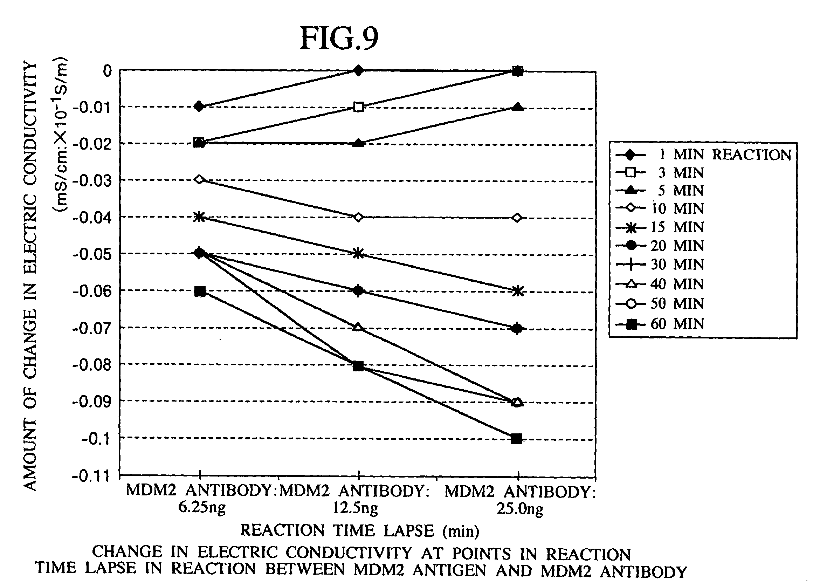

Measured values of electric conductivity at points in time lapse of a physiological saline in a reaction between an MDM2 antigen and a standard MDM2 antibody are illustrated in Fig. 8. In Fig. 8, the measured values of electric conductivity are represented by amounts of changes in electric conductivity from the electric conductivity of the physiological saline. Amounts of changes in electric conductivity for each MDM2 antibody concentration are shown in Fig. 9.

As is known from Figs. 8 and 9, an immune complex was generated from the antigen-antibody reaction between the MDM2 antigen and the standard MDM2 antibody reactive specifically with the MDM2 antigen, and a decrease in electric conductivity was observed with the lapse of time. The amount of change in electric conductivity with the lapse of time is larger according as the amount of MDM2 antibody is larger. For a certain amount of MDM2 antigen, concentration-dependency of MDM2 antibody was detected.

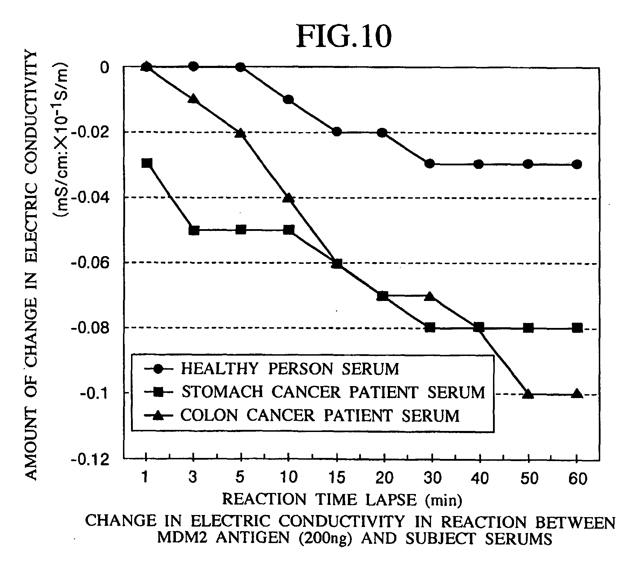

Fig. 10 illustrates measured values of electric conductivity at points in time lapse when a subject serum of a healthy person, and sera of cancer patients (a stomach cancer patient and a colon cancer patient) are added to a physiological saline containing MDM2 antigen. In Fig. 10, measured values of electric conductivity are represented by amounts of changes from the electric conductivity of the physiological saline.

As is clear from Fig. 10, there is observed an evident difference in time-dependent change of electric conductivity in the reaction with the MDM2 antigen between the healthy person subject serum and the subject sera of the cancer patients (a stomach cancer patient and a colon cancer patient).

By thus time-dependently measuring electric conductivity of the reaction solution, it is possible to confirm the presence of an MDM2 autoantibody existing in human blood. By comparing values of reactivity per lapse of time in the reaction between the subject sera and the MDM2 antigen, it is also possible to easily detect a clear difference the healthy person and the cancer patients. As a result, by comparing with a prescribed threshold value (cutoff value), it is possible to easily evaluate in a real-time manner the amount of MDM2 autoantibody in the sample. As a matter of course, as in Example 3, the MDM2 autoantibody in the subject sera can be quantitatively determined further in detail by using a prescribed calibration curve.

For example, the relationship between the amount of MDM2 antibody and the amount of change in electric conductivity is obtained from Fig. 9 representing the MDM2 antibody concentration on the abscissa and change (negative value) in electric conductivity on the ordinate regarding the result shown in Fig. 8. As in Example 3, this relationship can be used, for example, as a calibration curve for quantitative determination of the MDM2 autoantibody, as representing dependency of the reaction between the MDM2 antigen and the standard MDM2 upon concentration of the standard MDM2 antibody. It is known from the calibration curve at 60 minutes of reaction that the amount of the MDM antibody in the serum of the stomach cancer patient is about 6.25 µg/ml, and the amount of MDM antibody in the serum of the colon cancer patient is about 12.5 µg/ml. On the other hand, the amount of MDM antibody for the healthy person is smaller than that for the cancer patients: about 3 µg/ml or smaller.

According to the invention, as is clear from the description of Examples 3 and 4, it is possible to very easily detect and quantitatively determine a specific substance in a sample by measuring electric conductivity of an electrolytic solution. It is therefore possible to very easily and rapidly detect and quantitatively determine substances relating to a specific state of a disease present in the sample such as cancer-related substances, cancer-related gene products and an antibody produced against them.

Therefore, by easily and rapidly detecting and quantitatively determining substances relating to a specific status of a disease, such as the cancer-related substances, cancer-related gene products, or antibodies thereagainst existent in a sample such as human serum, the present invention is very useful in various clinical stages including diagnosis, inspection and establishment of a therapeutic indicator against cancer.

A specific substance can be detected and quantitatively determined by adding a reactant directly to the electrolytic solution in the invention. It is therefore possible to reduce waste, and detect and quantitatively determine a specific substance in the sample by means of a very simple apparatus.

Example 5

As another example of reaction of substances in an electrolytic solution, detection of a reaction of two substances, in which one of such substances is decomposed into smaller reaction products will now be described.

In this example, an enzymatic digestive reaction of a K1 antibody to which a K1 antibody and an enzyme pepsin is pertain are detected. In this example, the reaction to be detected was an enzymatic reaction.

A pepsin originating from hog stomach mucosa (3,520 Units/mg protein; available from SIGMA Company) (molecular weight: about 34,700) was used.

Measuring procedure

An amaunt of 10 ml of physiological saline was provided in a small-capacity vial (capacity: 12 ml), and a cell was immersed in this vial. An amount of 20 $\mu$l (absolute amount: 50 ng) of K1 antibody adjusted with physiological saline to a concentration of 2.5 $\mu$ g/ml was added into this vial by means of a microsyringe and stirred. Subsequently, pepsin adjusted with physiological saline to a concentration of 1 mg/ml (3,520 Units/mg protein) was added to this solution by means of a microsyringe in an amount of 5 $\mu$l (i.e., 17.6 Units/5 $\mu$ g protein). After stirring this reaction solution, electric conductivity was time-dependently measured while keeping the cell immersed in the reaction solution.

Result

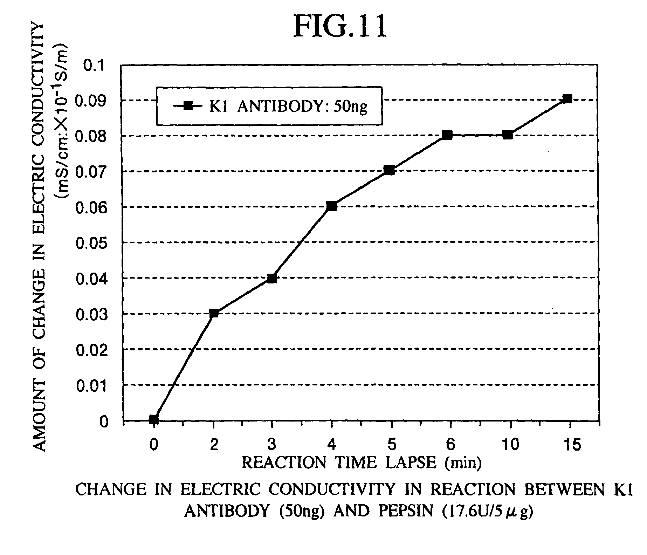

Measured values of electric conductivity at points of lapse of reaction time in an enzymatic digestion reaction between K1 antibody and pepsin are shown in Fig. 11. In Fig. 11, measured values of electric conductivity are represented by amounts of change in electric conductivity obtained by using the electric conductivity immediately after addition of pepsin as a blank value, and subtracting the blank value from values of electric conductivity at points of time lapse.

As shown in Fig. 11, electric conductivity showed a larger value with the lapse of reaction time.

This pepsin is known to cut the 234-th and 333-th amino residues of the H-chain of immunoglobulin IgG, and generate F(ab')2 and pFc' (molecular weight: about 100,000 and about 50,000, respectively) fragments. It is also known that on further causing pepsin to act, a peptide having lower molecular weight may be generated.

In this example, therefore, increasing in electric conductivity along with the lapse of reaction time demonstrates that the original K1 antibody was decomposed into smaller pieces of peptide through the enzymatic digestion of K1 antibody, and this made it easier for ions to move in the electrolytic solution with the lapse of reaction time.

According to the invention, as described above, it is possible to detect the time-dependent status of reaction and reaction products by measuring electric conductivity of the electrolytic solution, even when the decomposition reaction generates smaller reaction products.

By using a substance generating decomposition products on specifically decomposing a specific substance, almost as in the above-mentioned Example 2, it is possible to detect a specific substance, i.e., confirm the presence thereof, or evaluate and quantitatively determine the amount thereof. Vice versa, by using a specific substance which generates decomposition products on being decomposed specifically by a specific substance, it is possible to detect the specific substance causing a decomposition reaction, or evaluate and quantitatively determine the amount thereof.

As is known from this example, as described above, it is possible to detect the time-dependent reaction status of an enzyme reaction and reaction products by measuring electric conductivity of the electrolytic solution. According to the invention, therefore, it is possible to very easily and rapidly detect the reaction of substances (time-dependent reaction status and/or reaction products) in an electrolytic solution, irrespective of the kind of reaction or reactants, and to detect and quantitatively determine specific substances in a sample.

In the aforementioned Examples l to 5, electric conductivity was measured by means of an electric conductivity meter (CM30V digital electric conductivity meter made by TOA Electronics Ltd. (DKK · TOA Corporation at present)) with simultaneous use of an automatic temperature compensation (ATC) function automatically correcting a change in electric conductivity caused by a change in solution temperature, by measuring temperature of the subject solution.

More specifically, electric conductivity of the solution varies with temperature: a higher temperature leads to a higher electric conductivity, and a lower temperature leads to a lower electric conductivity. In order to compare values of electric conductivity irrespective of the actual temperature of the subject solution, therefore, it is the usual practice to convert a measured value into a value of electric conductivity at a certain temperature (reference temperature). The conversion formula is as follows:

$$\kappa_{REF} = \kappa_t/[1+(\alpha/100)(t - t_{REF})]$$

where,

$\kappa_{REF}$: Electric conductivity converted for reference temperature (S/m);

$\kappa_t$: Electric conductivity at t°C (S/m);

$\alpha$: Temperature coefficient (%/°C);

$t_{REF}$: Reference temperature (°C).

At a reference temperature of 25°C, the temperature coefficient is about 2% for most aqueous solutions. A measured value of electric conductivity is therefore usually converted automatically into a value of electric conductivity at the reference temperature (25°C), by setting the default temperature coefficient at 2%/°C and measuring temperature of the subject solution by means of a temperature sensor (thermistor) built, for example, in the cell (automatic temperature compensation (ATC)). A temperature coefficient may be manually set in response to the subject solution. It is of course also possible to measure temperature of subject solution separately, and conduct the temperature correction manually with reference to a prescribed temperature coefficient at a prescribed reference temperature. In the aforementioned Examples, the temperature correction was performed under conditions including a reference temperature of 25°C and a temperature coefficient of 2%, through the above-mentioned automatic temperature compensation (ATC).

Electric conductivity is not primarily measured in a state without temperature compensation.

However, as described later in detail, possibility was found to more accurately detect a reaction between substances, particularly an immune reaction between an antigen and an antibody, by measuring electric conductivity of a reaction solution without conducting temperature correction on turning OFF the automatic temperature compensation (ATC) of the electric conductivity meter, in a state in which temperature of the reaction solution is free from the effect of temperature of external atmosphere outside the reaction system (the temperature of external atmosphere is made constant, or exchange of heat between the reaction system and the external atmosphere outside the reaction system is cut off, or the reaction system and the external atmosphere outside the reaction system are kept at the same temperature). This point will now be described in detail with reference to some examples.

Example 6

The temperature coefficient of the electrolytic solution (physiological saline: an aqueous NaCl solution of 0.15 M) used commonly in the examples was determined.

A cell is immersed in 10 ml physiological saline in a small-capacity vial (capacity: 12 ml). This vial, together with the contents, was immersed in a water bath, and temperature was slowly reduced from 27°C. Without using an automatic temperature compensation (ATC) of the electric conductivity meter, or more specifically, in a state in which temperature correction was not substantially performed by setting the temperature coefficient at 0.00%/°C, measured value of temperature detected by a thermistor built in the cell and measured value of electric conductivity were time-dependently recorded to measure changes in electric conductivity relative to a change in solution temperature.

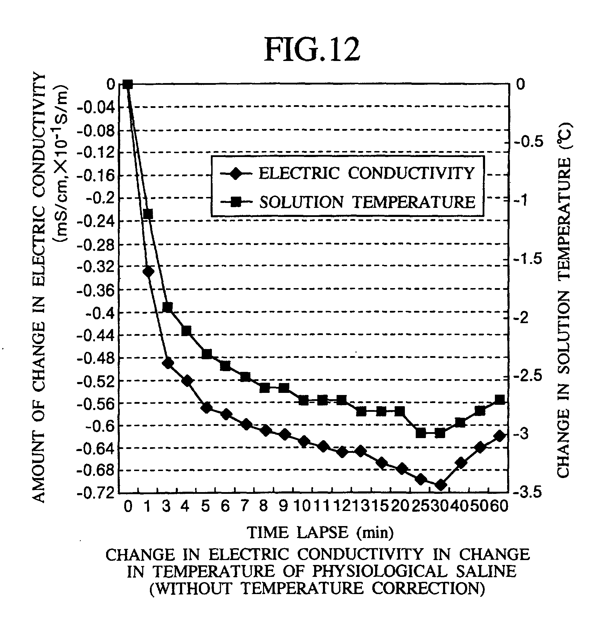

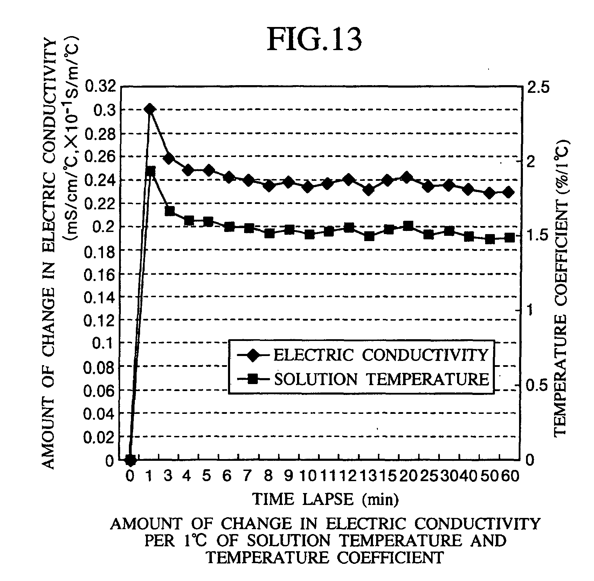

The result is shown in Fig. 12. In Fig. 12, measured values of electric conductivity and solution temperature are represented by amounts of change in electric conductivity and in solution temperature obtained by using the electric conductivity and the solution temperature at the start of measurement (time lapse: 0 minute) as blank values, and subtracting the blank values from the electric conductivity values and the solution temperature values at points in time lapse. From the result of measurement shown in Fig. 12, a change in electric conductivity per 1°C of solution temperature (mS/cm/1°C, x 10$^{-1}$S/m/1°C) and a rate of change of electric conductivity per 1°C of solution temperature (hereinafter referred to as "temperature coefficient") (%/1°C), at points in time lapse were determined, and the results are shown in Fig. 13.

As is understood from the result shown in Fig. 12, electric conductivity varies in parallel along with a change in temperature of the electrolytic solution. The result shown in Fig. 13 reveals that the average of temperature coefficient values (%/1°C) at points in time lapse is 1.56%, and the temperature coefficient showed almost a constant value irrespective of the time lapse.

Then, for a reaction similar to that in Example 1, i.e., for an antigen-antibody reaction between standard BFP antigen and K1 antibody, the time-dependent reaction status and a reaction product (immune complex) were detected without using the automatic temperature compensation (ATC) of the electric conductivity meter.

Measuring procedure

The measuring procedure as in Example 1 was applied except for nonuse of automatic temperature compensation (ATC). More specifically, an amount of 2 µl (absolute amount: 0.5 ng) of K1 antibody was added by use of a microsyringe to a physiological saline. An amount of 2 µl (absolute amounts: 0.6 ng, 1.2 ng and 2.4 ng) of standard BFP antigen was added to this solution. After stirring the resultant reaction solution, electric conductivity and solution temperature were time-dependently measured while keeping a cell immersed in the reaction solution.

Also for physiological salt solutions each containing singly the standard BFP antigen (absolute amount: 4.8 ng) and the K1 antibody (absolute amount: 1 ng), electric conductivity and solution temperature were time-dependently measured.

In order to avoid the influence of temperature of external atmosphere on the temperature of the reaction solution, the reaction was caused in an isothermal room (26°C) capable of keeping a constant room temperature.

Result

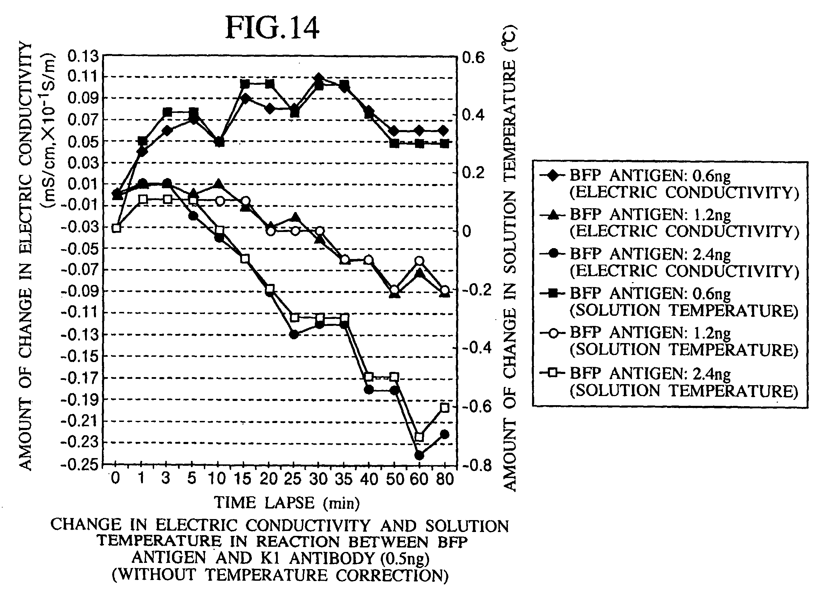

Measured values of electric conductivity and solution temperature at points in time lapse of the reaction between the standard BFP antigen and the K1 antibody are shown in Fig. 14. In Fig. 14, measured values of electric conductivity and solution temperature are represented by amounts of change in electric conductivity and solution temperature obtained by using values of electric conductivity and solution temperature at the start of reaction as blank values, and subtracting the blank values from values of electric conductivity and solution temperature at points in time lapse.

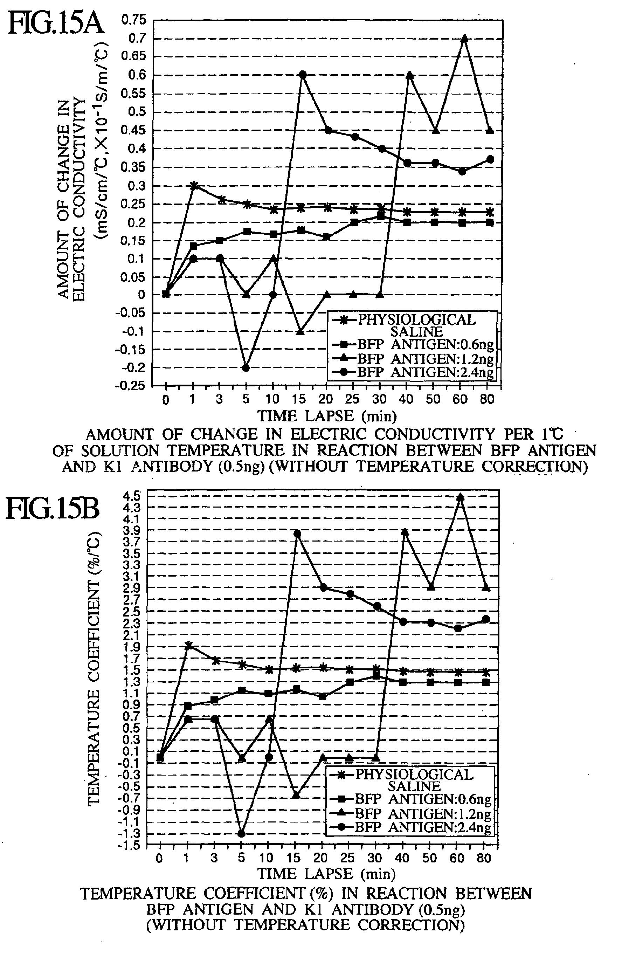

From the result shown in Fig. 14, a change in electric conductivity per 1°C of solution temperature (mS/cm/1°C) and a rate of change of electric conductivity per 1°C of solution temperature (temperature coefficient (%/1°C)) in each run of reaction were determined, and are shown in Figs. 15A and 15B, respectively.

As is understood from Fig. 14, in the reactions between the standard BFP antigen of the individual concentration and the K1 antibody, there is a good correlation between the change in electric conductivity and the change in solution temperature. For the K1 antibody (0.5 ng), a larger amount of standard BFP antigen led to further larger change (decrease) in electric conductivity and solution temperature.

As is known from Fig. 15A, with an amount of standard BFP antigen of 0.6 ng, the change in electric conductivity per 1°C of solution temperature (mS/cm/1°C) was approximately equal to that of the physiological saline. With an amount of standard BFP antigen of 1.2 ng, in contrast, a large change (increase) exceeding that of the physiological saline was observed after the lapse of 40 minutes. With an amount of standard BFP antigen of 2.4 ng, furthermore, a large change (increase) in electric conductivity was observed after the lapse of 15 minutes.

As described above, the amount of change in electric conductivity per 1°C of solution temperature (mS/cm/1°C) was found to largely vary between reactions to which difference amounts of BFP antigen are pertain, not uniform as in the case with electrolytic solution atone. The temperature coefficient (%/1°C) is a calculated value of the ratio of the amount of change in electric conductivity (mS/cm/1°C) per 1°C of solution temperature at points in time lapse relative to electric conductivity of the reaction solution before reaction, and the result exhibits the same tendency as the amount of change in electric conductivity (mS/cm/1°C). That is, the temperature coefficient (%/1°C) was found to time-dependently vary with the amounts of antigen and antibody in an immune reaction.

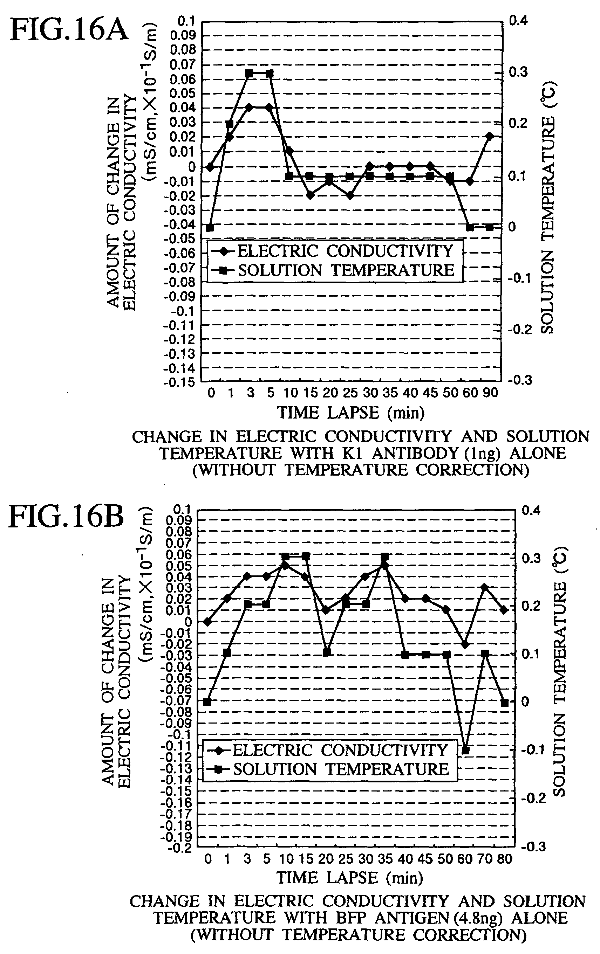

On the other hand, measured values of electric conductivity and solution temperature at points in time lapse of physiological salt solutions singly containing K1 antibody or standard BFP antigen are shown in Figs. 16A and 16B, respectively. In Fig. 16, measured values of electric conductivity and solution temperature are represented by amounts of change in electric conductivity obtained by using the electric conductivity at the start of reaction as a blank value, and subtracting the blank value from values of electric conductivity at points in time lapse.

As is clear from Figs. 16A and 16B, no significant change in electric conductivity and solution temperature was observed for both the standard BFP antigen and the K1 antibody. From this result, it is evident that changes in electric conductivity and solution temperature are caused by the reaction between BFP antigen and K1 antibody.

Example 7

A reaction between BFP antigen and K1 antibody in which a protein (fetal calf serum (FCS)) was added to a reaction system, as in Example 2, was detected without using automatic temperature compensation (ATC) of an electric conductivity meter.

Measuring procedure

The measuring procedure was the same as in Example 2 except that automatic temperature compensation (ATC) was not used. More specifically, an amount of 2 μl (about 140 μg protein in absolute amount) of fetal calf serum (FCS) (about 70 mg protein/ml) was previously added to a physiological saline. For each reaction between K1 antibody (0.5 ng) and standard BFP antigen of different concentrations (0.075 ng and 0.15 ng), electric conductivity and solution temperature were time-dependently measured without using automatic temperature compensation (ATC) of an electric conductivity meter. As in Example 2, the fetal calf serum was added for the purpose of achieving an amount of protein corresponding to the amount added to the reaction system upon evaluating and quantitatively determining the amount of BFP antigen in the subject serum in an example described later. As in Example 6, the reaction was conducted in an isothermal room (26°C).

Result

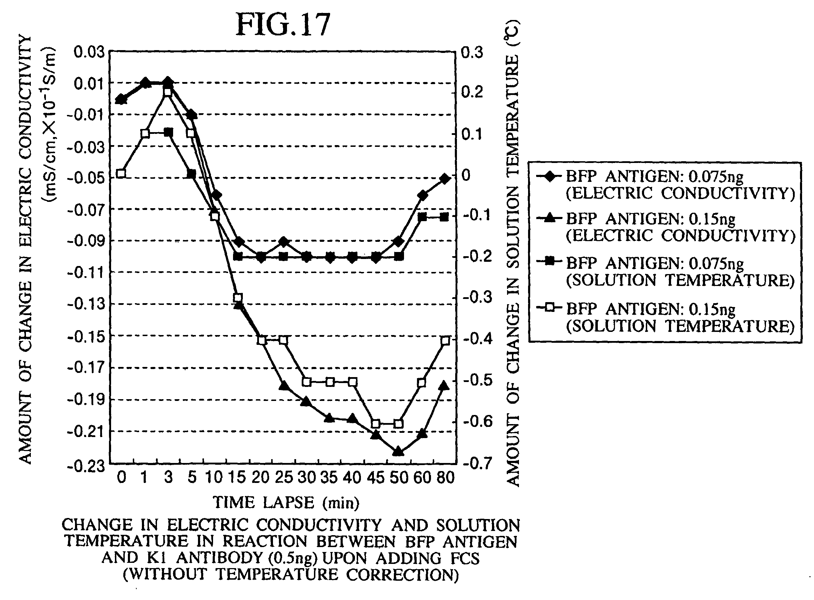

Measured values of electric conductivity and solution temperature at points in time lapse of reaction between BFP antigen and K1 antibody are shown in Fig. 17. In Fig. 17, measured values of electric conductivity and solution temperature are represented by amounts of change in electric conductivity and solution temperature obtained by using values of electric conductivity and solution temperature at the start of reaction as blank values, and subtracting the blank values from values of electric conductivity and solution temperature at points in time lapse.

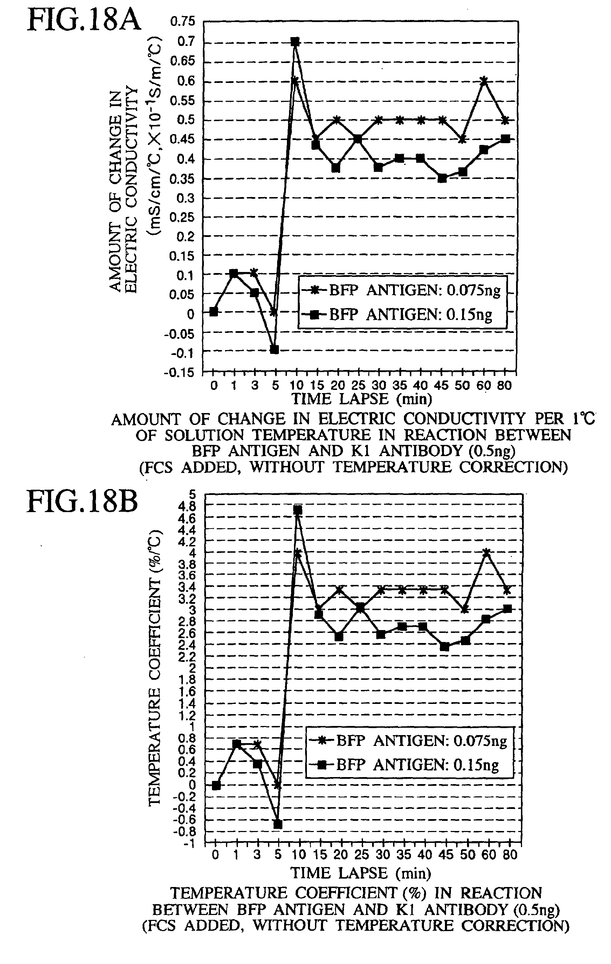

From the result shown in Fig. 17, a change in electric conductivity per 1°C of solution temperature (mS/cm/1°C) and a rate of change of electric conductivity per 1°C of solution temperature (temperature coefficient (%/1°C) in the individual reactions were determined from the result shown in Fig. 17, are shown in Figs. 18A and 18B, respectively.

As is understood from the result shown in Fig. 17, there is a good correlation between the change in electric conductivity and the change in solution temperature in reactions between standard BFP antigens of the inivisual concentration and K1 antibody. With a larger amount of standard BFP antigen relative to the K1 antibody (0.5 ng), larger changes were observed in electric conductivity and solution temperature.

As is known from Fig. 18A, both the amount of change in electric conductivity per 1°C of solution temperature (mS/cm/1°C) and the temperature coefficient (%/1°C) showed no marked difference in time-dependent change between the two levels of amount of antigen. These values, however, largely varies after the lapse of ten minutes of reaction: the temperature coefficient (%/1°C) was, for example, about 3% which is considerably larger than that of the physiological saline of 1.56%.

In this example, it was possible to grasp the reactions between very slight amounts of the standard BFP antigen as 0.075 ng or 0.15 ng and the K1 antibody (0.5 ng). Not intending to be bound by only a particular theory, this is attributable to the fact that addition of fetal calf serum (FCS) to the reaction system permitted maintenance of stability of the antigen in a slight amount in the electrolytic solution, and the detecting sensitivity of antigen-antibody reaction was improved.

As is known from the result shown in Figs. 18A and 18B, the reaction of the standard BFP antigen with the K1 antibody (0.5 ng) is on almost the same level for two cases of the amount of standard BFP antigen of 0.075 and 0.15 ng. A difference in reactivity was observed between the reaction of such a small amount of standard BFP antigen with the K1 antibody, and the reaction in which the amount of standard BFP antigen of 0.6 to 2.4 ng, as shown in Figs. 14, 15A and 15B.

Example 8

As in Example 3, a BFP antigen in a serum was detected as a specific substance in a sample, without using automatic temperature compensation (ATC) of the electric conductivity meter.

Human sera of a healthy person and a cancer patient (hepatoma) freeze-stored at -20°C were used as subject sera. The amount of BFP antigen in sera of the healthy person and the cancer patient used in this example was 35 ng/ml and 100 ng/ml, respectively, as measured by use of "Lanazyme (trade mark) BFP Plate" of Nippon Kayaku Co., Ltd.

Measuring procedure

The measuring procedure was the same as that in Example 3 except that automatic temperature compensation (ATC) was not used. More specifically, an amount of 2 µl (absolute amount: 0.5 ng) of K1 antibody was added by means of a microsyringe to 10 ml physiological saline, and the solution was stirred. An amount of 2 µl (about 140 µg protein in absolute amount) of subject serum (about 70 mg protein/ml) brought back to room temperature was added by means of a microsyringe to the resultant solution, and stirred. Electric conductivity and solution temperature were time-dependently measured, without using automatic temperature compensation (ATC) while keeping a cell immersed in this reaction solution. As in Examples 6 and 7, the reaction took place in an isothermal room (26°C).

Reactions were caused for the serum of the cancer patient under the same conditions on varying the concentration of K1 antibody (absolute amounts: 0.0625 ng, 0.25 ng and 0.5 ng), and electric conductivity and solution temperature were time-dependently measured, similarly without using automatic temperature compensation (ATC) of the electric conductivity meter.

Result

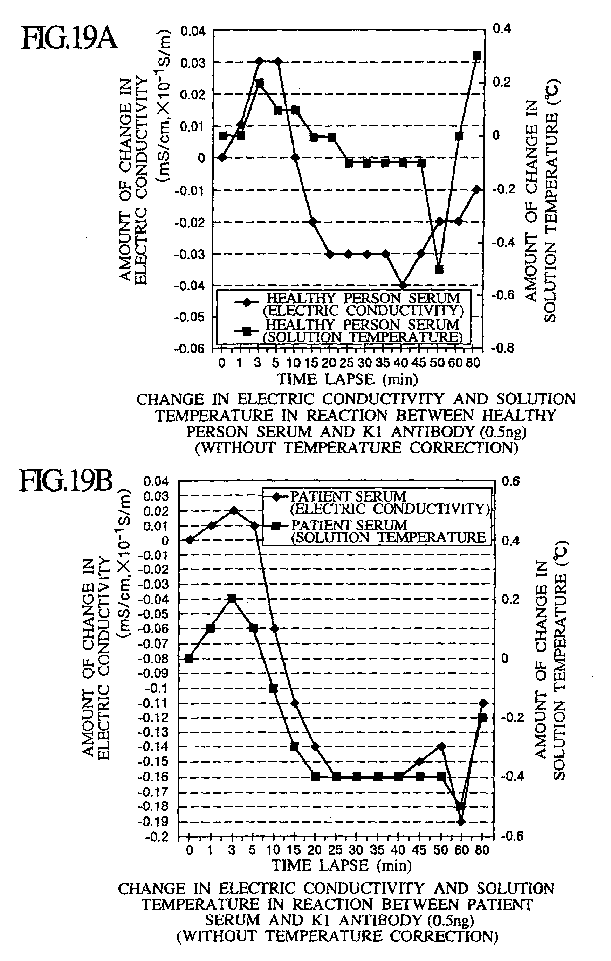

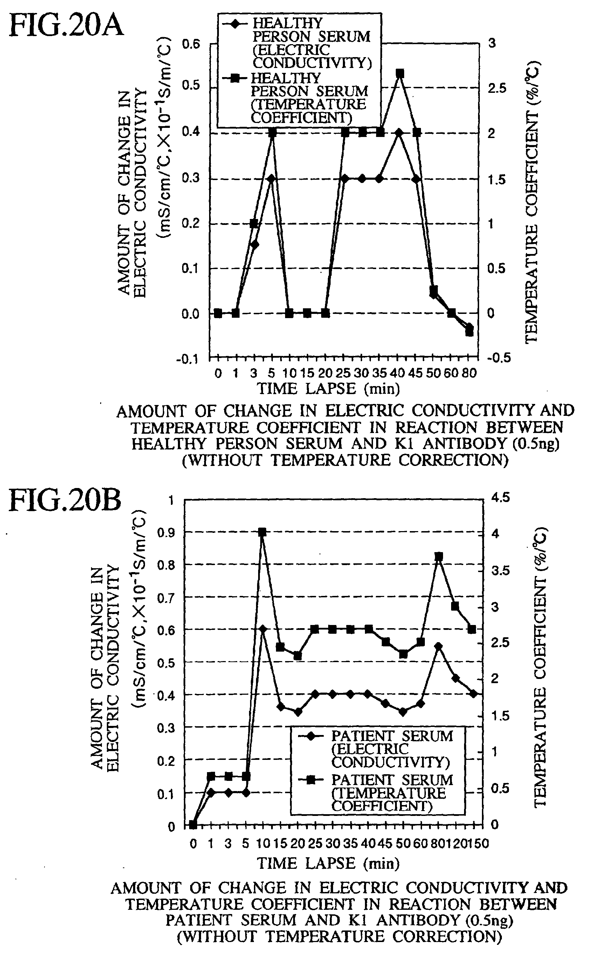

For each of the reactions of the healthy person serum and the cancer patient serum with the K1 antibody, measured values of electric conductivity and solution temperature at points in time lapse are shown in Figs. 19A and 19B. Changes in electric conductivity per 1°C of solution temperature (mS/cm/1°C) and rates of change of electric conductivity per 1°C of solution temperature (temperature coefficient (%/1°C)) were determined from the result shown in Figs. 19A and 19B for each reaction. The result is shown in Figs. 20A and 20B, respectively.

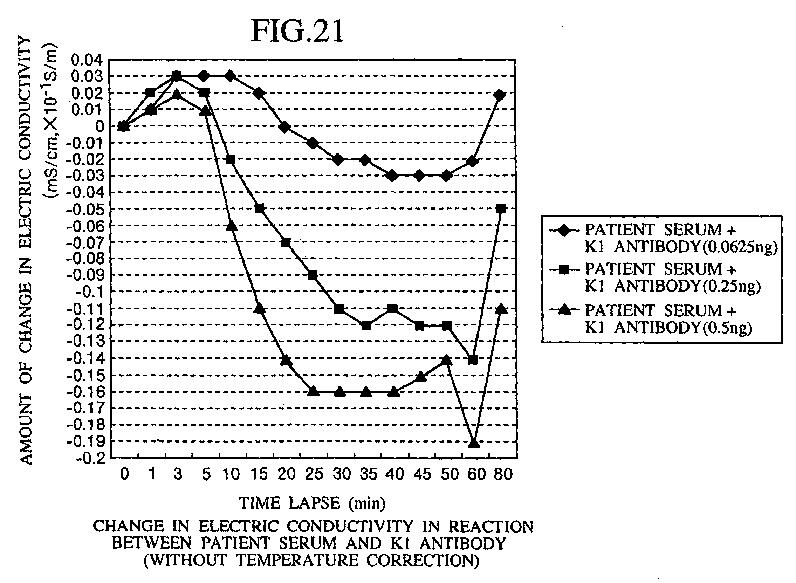

Measured values of electric conductivity and solution temperature at points in time lapse for each of the reactions of K1 antibodies of three kinds of concentrations with the cancer patient serum are shown in Fig. 21.

In Figs. 19 and 21, measured values of electric conductivity and solution temperature are represented by amounts of change in electric conductivity and solution temperature obtained by using values of electric conductivity and solution temperature at the start of reaction as blank values, and subtracting the blank values from values of electric conductivity and solution temperature at points in time lapse.

A decrease in electric conductivity accompanied by the decrease in solution temperature was observed in both the healthy person serum and the cancer patient serum in the result shown in Figs. 19A and 19B, as in the reaction between the BFP antigen and the K1 antibody in Example 7. As compared with the healthy person serum, a larger change in electric conductivity was observed in the cancer patient serum.

The amount of change in electric conductivity per 1°C of solution temperature (mS/cm/1°C) and the temperature coefficient (%/1°C) are different between the healthy person and the cancer patient. Those of the healthy person was known to be smaller that those of the cancer patient. For the cancer patient serum, the amount of change in electric conductivity (mS/cm/1°C) and the temperature coefficient (%/1°C) varied largely upon the lapse of ten minutes after the start of reaction, and were almost constant until the lapse of 60 minutes. The value thereof showed a progress of 2.3 to 4 (%/1°C) which is higher than the temperature coefficient of the electrolytic solution of 1.56 (%/1°C).

It is thus possible to detect an immune reaction of the sera of a healthy person and a cancer patient with K1 antibody by measuring electric conductivity, and time-dependently compare reactivity. The behavior relative to the K1 antibody is evidently different between the healthy person serum and the cancer patient serum.

In the reaction between the standard BFP antigen (0.075 ng or 0.15 ng) and the K1 antibody (0.5 ng) when adding fetal calf serum (FCS) to the reaction system shown in Example 7, the amount of BFP antigen in the reaction solution of 0.075 ng, if converted, corresponds to an amount of BFP antigen in serum of 37.5 ng/ml, and the amount of BFP antigen in the reaction solution of 0.15 ng, if converted, corresponds to an amount of BFP antigen in serum of 75 ng/ml.

Therefore, collation of the reactivity of the healthy person serum or the cancer patient serum relative to the K1 antibody in this example with each reaction in the Example 7 suggests the presence of the BFP antigen corresponding to 37.5 ng/ml or less in the healthy person serum. In the cancer patient serum, on the other hand, the BFP antigen corresponding to approximately 75 ng/ml is considered to be present.

As described above, it is known, by the application of the EIA method, that BFP antigen is present in amounts of 35 ng/ml and 100 ng/ml, respectively, in the healthy person serum and the cancer patient serum used in this example. Regarding the amount of BFP antigen contained in the sera, the estimated value in this example is almost of the same order as the measured value by the EIA method. As in Example 3, it is of course possible to quantitatively determine BFP in a subject serum further in detail by using a prescribed calibration curve. For example, it is possible to obtain a relationship between the amount of standard BFP antigen and the amount of change in electric conductivity as shown in Figs. 22A and 22B, by plotting, from the results shown in Figs. 14 and 17, values of standard BFP antigen concentration on the abscissa, and values of changes in electric conductivity (negative values) on the ordinate. This relationship can be used as a calibration curve.

As shown in Fig. 21, a change in electric conductivity depending upon the K1 antibody concentration was observed in the cancer patient serum. It is considered from this result that a reaction property in which serum BFP antigen in an amount meeting the amount of the K1 antibody is reacted with the K1 antibody was observed through measurement of electric conductivity.

As is clear from the result of experiment shown in Examples 6 to 8, on measuring electric conductivity and solution temperature in a state in which temperature correction is not applied without using automatic temperature compensation (ATC) of an electric conductivity meter, there is apparently observed a decreasing tendency of solution temperature according as an antigen-antibody reaction in an immune reaction proceeds. Electric conductivity was found to become lower along with this decrease in temperature.

As described above, electric conductivity of the solution varies with temperature: a higher temperature leads to a higher electric conductivity, and a lower temperature results in a lower electric conductivity. However, as is evident from Figs. 15A and 15B, the amount of change in electric conductivity per 1°C of solution temperature (mS/cm/1°C) and the temperature coefficient (%/1°C) time-dependently vary with each reaction of different amounts of standard BFP antigen. Therefore, a change in electric conductivity in an immune reaction is not dependent only on a decrease in solution temperature, but is considered to reflect the result brought about by the synergetic effect with the increase in electric resistance along with the progress of the immune reaction, i.e., formation of the immune complex.

The above-mentioned point will be described further in detail. Not intending to be bound by a particular theory, in a reaction between substances, particularly in an immune reaction, a binding energy is required upon reaction of an antigen and an antibody, and this is considered to cause a decrease in temperature of the reaction solution. Along with this decrease in solution temperature, electric conductivity of the electrolytic solution varies. However, this change in electric conductivity caused by a change in solution temperature in response to the status of reaction exceeds the range of change in electric conductivity caused only by a change in solution temperature of the electrolytic solution. That is, as described above, binding of the antigen and the antibody generates larger molecules (immune complex), and this makes it difficult for electricity to flow, and this is considered to cause a decrease in electric conductivity. As a result, this is considered to more accurately reflect the reaction between substances.

According to a study carried out by the present inventor, when the antigen or the antibody has a lower concentration, the change in electric conductivity depends upon the decrease in temperature of the reaction solution caused by an immune reaction, and when the concentration is higher, the degree of contribution of the increased electric resistance resulting from formation of larger molecules is considered to be increased.

In other words, as is known from the results shown in Figs. 15A, 15B, 18A, 18B, 20A and 20B, when the amount of the standard BFP antigen is small relative to the K1 antibody of a constant amount in an immune reaction between standard BFP antigen and K1 antibody, the amount of change in electric conductivity per 1°C of solution temperature (mS/cm/1°C) and the temperature coefficient (%/°C) is generally equal to those of the physiological saline, and these values become larger than those of the physiological saline according as the amount of BFP antigen increases and reactivity becomes higher.

According to the method explained in Examples 1 to 5, there is available a very remarkable effect as described above. However, in measurement using automatic temperature compensation (ATC) of an electric conductivity meter, a constant temperature compensation is conducted for a change in temperature of the reaction solution. It is therefore probable that a result sufficiently reflecting the original reaction is not available. In order to accurately grasp the original reaction, therefore, it would be desirable that a change in solution temperature and a change in electric conductivity caused by the reacting substances can be accurately grasped. For this purpose, it is essential that the reaction solution temperature is not affected by the temperature, for example, of surroundings.

In order to accurately detect a reaction of substances by measuring electric conductivity of the reaction solution, therefore, it would be preferable to achieve a state in which it is possible to accurately measure a change in solution temperature caused by the original reaction while ensuring that the temperature of the reaction solution is free from the effect of, for example, the outside open air temperature, and to exclude automatic temperature compensation of electric conductivity in this state, that is, to measure electric conductivity as well as solution temperature without performing temperature correction.

A state in which temperature of the reaction solution is free from the effect of temperature outside the reaction system such as open air temperature can be achieved by adopting any one or a combination of means for maintaining the atmosphere outside the reaction system in which the reaction of substances takes place in the electrolytic solution at a constant temperature, means for thermally shielding the reaction system from the external atmosphere, and means for maintaining the reaction system and the external atmosphere at the same temperature, i.e., means for causing the external atmosphere temperature of the reaction solution to vary in response to a change in reaction solution temperature and eliminating heat input and output substantially between the reaction solution and outside the solution.

In the above-mentioned Examples 6 to 8, the reaction is conducted by placing a reactor (vial) and an electric conductivity meter in an isothermal room keeping a constant temperature so that the influence of temperature of external atmosphere is not exerted on temperature of the reaction solution, and electric conductivity and solution temperature were measured.

Comparison of the results shown in Fig. 5 (Example 2) and Fig. 17 (Example 7), or shown in Fig. 7 (Example 3) and Fig. 19A (Example 8) reveals that reaction products and status of reaction can be detected more sensitively by measuring electric conductivity without using automatic temperature compensation (ATC) of the electric conductivity meter.

As is known from the result shown in Examples 6 to 8, it is possible to very easily detect reaction products (immune complex) from reaction of substances, and status of reaction (reaction properties) between substances including dependency of reactivity upon concentration of antigen or antibody from the amount of change in electric conductivity per 1°C of solution temperature (mS/cm/1°C) or the temperature coefficient (%/1°C), by time-dependently measuring electric conductivity and solution temperature without using automatic temperature compensation (ATC) of the electric conductivity meter. Furthermore, by comparing, for example, the amount of change in electric conductivity (mS/cm/1°C) or the temperature coefficient (%/1°C), it is possible to detect specific substances in a sample, or evaluate and quantitatively determine the amount thereof.

As a matter of course, the advantage of measuring electric conductivity by use of a common temperature correcting technique such as automatic temperature compensation (ATC) described above is always remarkable in that a reaction of substances can be very easily measured on the basis of measurement of electric conductivity of the reaction solution, without providing any special means so as to avoid the effect of the external atmosphere temperature outside the reaction system on the reaction solution temperature.

Example 9

Still another example of the invention will now be described. As described above, the present inventor obtained the following novel findings. When a state is achieved in which the reaction solution temperature is not affected by the external atmosphere temperature outside the reaction system, the reaction solution temperature decreases along with the progress of reaction, in a reaction considered to require binding energy in a reaction solution such as an immune reaction between antigen and antibody.

A study carried out by the present inventor suggests that, in a low-concentration reaction, a change in temperature is predominant over a change in electric conductivity rather than a change in electric resistance of the reaction solution caused by the reaction products. More specifically, it is suggested that, when the concentration of the antigen and antibody is low, electric resistance of the antigen and antibody is originally low, so that a change in temperature of the reaction solution is predominant over a change in electric conductivity resulting from the antigen-antibody reaction, as compared with a change in electric resistance of the reaction products.

On the basis of such novel findings, in a state in which the reaction solution temperature is free from the influence of the external atmosphere temperature outside the reaction system, and particularly in a low-concentration reaction, it is possible to detect reaction products and the status of reaction in a reaction considered to require binding energy in a reaction solution, such as an immune reaction, by only measuring temperature of the reaction solution.

As described above, a state in which the temperature of the reaction solution is free from the effect of temperature outside the reaction system such as open air temperature can be achieved by adopting any one or a combination of means for maintaining the atmosphere outside the reaction system in which the reaction of substances, especially immune reaction between antigen and antibody, takes place in the electrolytic solution at a constant temperature, means for thermally shielding the reaction system from the external atmosphere, and means for maintaining the reaction system and the external atmosphere at the same temperature, i.e., by means for causing the external atmosphere temperature of the reaction solution to vary in response to a change in reaction solution temperature and eliminating heat input and output substantially between the reaction solution and outside the solution.

As described above, the result shown in Figs. 14 and 17 reveals an apparent difference in a change in solution temperature in accordance with the amount of standard BFP antigen in a reaction between standard BFP antigen and K1 antibody. As is understood from Figs. 19A and 19B, there is an evident difference in a change in solution temperature between the healthy person serum and the cancer patient serum.

Further, in a state in which temperature of the reaction solution is free from the influence of external atmosphere temperature outside the reaction system, there is a good correlation between a change in electric conductivity and a change in solution temperature resulting from an immune reaction. According to this example, therefore, there are available various functional effects as in detection of a reaction based on measurement of electric conductivity, as described in detail with reference to the aforementioned examples.

More specifically, according to this example, by time-dependently measuring temperature of the reaction solution, it is possible to very easily detect an immune reaction without the need of expensive large-scaled equipment. According to the present invention, for example, it is possible, not only to detect a finally formed immune complex as in the conventional immunological measuring methods (RIA method and EIA method), but also to time-dependently detect time-dependent status of an immune reaction, an antigen, an antibody and/or an immune complex.

Because an immune reaction between an antigen and an antibody can be time-dependently measured, it is possible, for example, to carry out selection of an antibody more reactive with an antigen or an antigen more reactive with an antibody, or measurement of presence or absence of sensitivity between the antigen and the antibody very easily, rapidly and in a real-time manner.

Further, it is not necessary to solidify the antigen or the antibody into a carrier or to conduct operations such as preparation of a labelled substance as in the conventional immunological measuring methods (RIA method and EIA method), but an immune reaction can be detected rapidly and easily by adding reactants directly to the reaction solution. It is hence possible to detect an immune reaction by means of a very simple apparatus without discharging waste of a labelled substance, a reagent such as a coloring agent (enzyme substrate), or a solidification carrier.

By measuring temperature of the reaction solution, it is possible to very easily detect a specific substance (antigen or antibody) in a sample. The amount of such a specific substance can quantitatively determined through use of a prescribed calibration curve regarding a change in solution temperature previously determined in the same system, or comparison of the solution temperature to a prescribed threshold value. It suffices to appropriately set a calibration curve or a threshold value, as in the case of electric conductivity, in response to the reaction, for example, by using the reaction rate (time-dependent rate of change of solution temperature such as the initial reaction rate) as an indicator.

It is therefore possible to very simply and rapidly detect and quantitatively determine substances relating to a specific state of a disease present in a sample, for example, a cancer-related substance, a cancer-related gene product, or a antibody produced against such a substance. By simply and rapidly detecting and quantitatively determining, for example, the above-mentioned cancer-related substance, cancer-related gene product, or an antibody against such a substance present in a sample such as a human serum as a substance relating to the specific state of a disease, the present invention is very useful in various clinical stages including diagnosis, inspection and establishment of a therapeutic indicator of cancer. Furthermore, because of the possibility to accomplish detection and quantitative determination of a specific substance by adding a reactant directly to the reaction system, it is possible to reduce waste and detect and quantitatively determined a specific substance in a sample by means of a very simple apparatus.

Temperature measuring means (such as a thermistor) permitting measurement of solution temperature at a high accuracy as 1/100°C is available.

Example 10

An example of the apparatus having a configuration for the application of the invention will now be described.

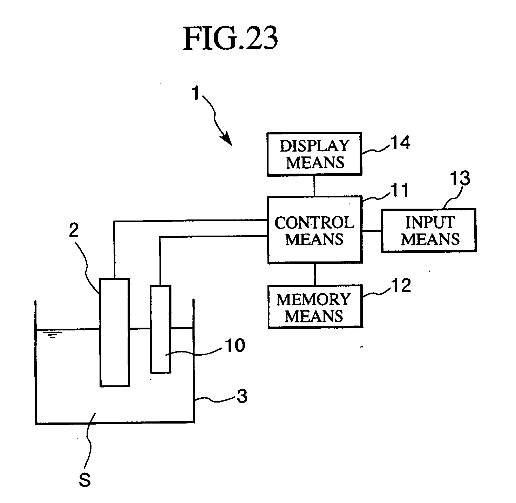

As shown in Fig. 23, the reaction detecting apparatus substantially comprises an electric conductivity meter 1 and a reactor (reactor vessel) 3.

The electric conductivity meter has a cell (electric conductivity measuring electrode pair) 2 which is electric conductivity detecting means, immersed in a subject solution S in the reactor 3 and issues a signal corresponding to the electric conductivity, control means 11 such as a microcomputer, which detects the signal issued by the cell 2 in response to a reaction in the subject solution S and processes data.

Memory means 12 may be connected to the control means 11. The control means 11 comprehensively controls apparatus operations in accordance with a program stored in the memory means 12, processes an output of the cell 2 on the basis of information stored in the memory means 12, and can thus generate a signal of a desired form in response to the electric conductivity of the subject solution S. Input means 13 may be connected to the control means 11. Setting of various parameters of the apparatus, start and stoppage of measurement, and inputting of desired data are performed on the input means 13. Further, display means 14 may be connected to the control means 11. A signal corresponding to the electric conductivity of the subject solution S generated by the control means 11 on the basis of an output of the cell 2 is transmitted to the display means 14, and can be displayed as a measuring result in a desired form. It is of course that a general-purpose computing/controlling device such as a personal computer can be applied as the control means 11, and ones connected to such a computer may be used as the memory means 12, the input means 13 and the display means 14.

Temperature detecting means 10 such as a thermistor for detecting temperature of the subject solution S may be provided in the apparatus 1. The temperature detecting means 10 may be built in the cell 2. A signal issued by the temperature detecting means 10 in response to temperature of the subject solution is entered into the control means 11. The control means 11 can display information about temperature of the subject solution on the display means 14 on the basis of an output of the temperature detecting means 10 corresponding to temperature of the subject solution.

As described above with reference to Examples I to 6, as is commonly done in the technical field of the present invention, the control means 11 can accomplish temperature correction of a measured value of electric conductivity based on the output of the cell 2, through automatic temperature compensation (ATC) or the like on the basis of an output of the temperature detecting means 10.

As described above with reference to Examples 6 to 8, on the other hand, in order to achieve a state in which temperature of the reaction solution is free from the influence of temperature outside the reaction system such as the open air temperature, means for maintaining the atmosphere outside the reactor 3 at a uniform temperature, means for thermally shielding the interior of the reactor 3 from the atmosphere out side the reactor 3, and means for maintaining the interior of the reactor 3 and the atmosphere outside the reactor 3 at the same temperature can be provided singly or in combination.

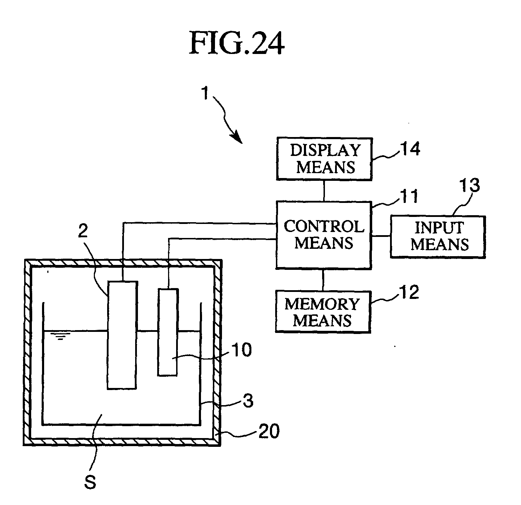

That is, the reactor 3, the cell 2 and at least the detecting section of the temperature detecting means 10 or the electric conductivity meter 1 itself may be arranged in an isothermal room. The medium for ensuring a uniform temperature for the atmosphere outside the reactor 3 may be any of a liquid, a solid, or a gas. Also, heat insulating means surrounding the reactor 3, the detecting section of the cell 2, and at least the detecting section of the temperature detecting means 10 may be provided. An appropriate heat insulating material or a vacuum vessel may be used as the heat insulating means. In addition, heat input/output between the subject solution in the reactor 3 and the outside may substantially be eliminated by causing a change in external atmosphere temperature of the subject solution in response to a change in temperature of the subject solution in the reactor 3. As an example, Fig. 24 illustrates a state in which the reactor 3, the cell 2 and the temperature detecting means 10 are surrounded by the heat insulating means 20.

As a result, it is possible to exclude the influence of surroundings such as temperature of external atmosphere on the reaction solution, and measure electric conductivity in a state without temperature correction such as automatic temperature compensation (ATC).

Further, in the configuration shown in Fig. 24, the control means 11 detects a signal issued by the temperature detecting means 10 in response to the reaction in the subject solution. Thus, it is possible to generate a signal corresponding to the amount of change in electric conductivity per 1°C of solution temperature or the temperature coefficient from this signal and the output signal of the cell 2.

In this configuration, the control means 11 can time-dependently detect and display electric conductivity and temperature of the subject solution S, and furthermore, detect specific substances in the subject solution (or evaluate and quantitatively determine the amount thereof) from an output of the cell 2, or from output of the cell 2 and the temperature detecting means 10, by us of prescribed threshold information and calibration curve information previously set in the memory means 12 or set via the input means. More specifically, by using, as an indicator, the time-dependent change in electric conductivity detected along with progress of the status of reaction by time-dependently measuring electric conductivity of the subject solution, or the amount of change in electric conductivity per 1°C of solution temperature (mS/cm/1°C) or the temperature coefficient (%/1°C) detected with progress of the reaction status (in the case where temperature of the subject solution corresponding to the reaction is time-dependently measured), it is possible to process data on the basis of threshold information or calibration curve information predetermined for specific substances contained in the subject solution. Regarding the method for detecting the specific substances in the subject solution, and the method for evaluating or quantitatively determining the amount thereof, , the description in Examples 1 to 8 is applied.

Example 11

An example of the immune reaction detecting apparatus for implementing the immune reaction detecting method described with reference to the above description of Example 9 will now be described.

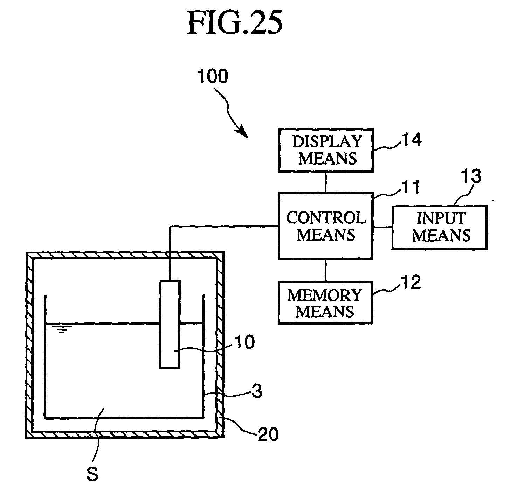

As shown in Fig. 25, the apparatus for application of the detecting method of an immune reaction described in Example 9 has a temperature detector 100 having a temperature detecting means 10 such as a thermistor, which substantially detects temperature of the subject solution S in the reactor 3.

In order to achieve a state in which temperature of the reaction solution is free from the influence of temperature outside the reaction system such as the open air temperature, means for maintaining the atmosphere outside the reactor 3 at a constant temperature, means for thermally shielding the interior of the reactor 3 from the atmosphere outside the reactor 3, and means for maintaining the interior of the reactor 3 and the atmosphere outside the reactor 3 at the same temperature can be provided singly or in combination.

That is, as in Example 10, the reactor 3, and at least the detecting section of the temperature detecting means 10 or the temperature detector 100 itself may be arranged in an isothermal room. The medium for ensuring a uniform temperature for the external atmosphere outside the reactor 3 may be any of a liquid, a solid, or a gas. Also, heat insulating means surrounding the reactor 3, and at least the detecting section of the temperature detecting means 10 may be provided. An appropriate heat insulating material or a vacuum vessel may be used as the heat insulating means. In addition, heat input/output between the subject solution in the reactor 3 and the outside may substantially be eliminated by causing a change in external atmosphere temperature of the subject solution in response to a change in temperature of the subject solution in the reactor 3. As an example, Fig. 25 illustrates a state in which the reactor 3, and the temperature detecting means 10 are surrounded by the heat insulating means 20.

In Fig. 25, the configuration comprising control means 11, memory means 12, input means 13, display means 14 and other components may be the same as in Example 10, except that an electric conductivity measuring cell 2 is not connected to the control means 11, and the control means 11 detects only a signal issued by the temperature detecting means 10 in response to the reaction in the subject solution and causes the display means 14 to display information corresponding to temperature of the subject solution 5. A detailed description is therefore omitted here.

In this configuration, the control means 11 can time-dependently detect and display temperature of the subject solution S, and furthermore, detect specific substances in the subject solution (or evaluate and quantitatively determine the amount thereof), by use of prescribed threshold information and calibration curve information previously set in the memory means 12 or set via the input means. More specifically, it is possible to process data on the basis of threshold information or calibration curve information predetermined for specific substances contained in the subject solution, by using, as an indicator, the change in solution temperature detected through progress of the reaction status by time-dependently measuring temperature of the subject solution. Regarding the method for detecting the specific substances in the subject solution, or the method for evaluating or quantitatively determining the amount thereof, the description in Example 9 is applicable.

Industrial Applicability

According to the present invention, as described above, it is possible to detect a reaction of substances more simply. Without the need for an expensive and large-scaled equipment or measuring instruments, it is possible to easily and rapidly detect time-dependent reaction status and/or reaction products of reactions of substances in a real-time manner. It is therefore very useful for detecting and quantitatively determining, for example, specific substances in a sample.

According to the invention, therefore, it is possible to more easily detect an immunological or enzymatic reaction. For example, it is permitted to more easily detect and quantitatively determine specific substances relating to a specific status of a disease. According to the invention, furthermore, it is possible to provide a new approach for easily and rapidly detecting time-dependent reaction status and/or reaction products of a reaction of various substances taking place in an electrolytic solution, by use of a simple apparatus.

In addition, according to the invention, it is possible to very simply detect an immunological reaction, and very easily and rapidly detect and quantitatively determine specific substances in a sample such as a specific substance relating to a specific status of a disease in a real-time manner.

**Claims**

1.  A reaction detecting method comprising detecting a reaction of a substance in an electrolytic solution on the basis of measurement of an electric conductivity of the electrolytic solution.

2. A reaction detecting method according to claim 1, wherein a reaction status and/or a reaction product of the reaction are detected by time-dependently measuring the electric conductivity of the electrolytic solution.

3. A reaction detecting method according to claim 1 or 2, wherein said reaction is (a) an immune reaction, (b) an enzyme reaction, or (c) any of the other chemical reactions including a bonding reaction, a polymerization reaction, a decomposition reaction and a catalytic reaction.

4. A reaction detecting method according to claim 1, 2 or 3, wherein the substance is (i) a protein including a purified protein and a synthetic protein, (ii) a enzyme, (iii) a antigen including (i) and (ii) above, (iv) an antibody including a polyclonal antibody and a monoclonal antibody, or (v) any of the other chemical substances.

5. A reaction detecting method according to any one of claims 1 to 4, wherein a specific substance in a sample is detected and/or quantitatively determined by detecting a reaction between the specific substance and a substance reactive with the specific substance as the reaction.

6. A reaction detecting method according to claim 5, wherein the specific substance is a substance associated with a specific symptom of a disease, a product of a gene associated with a specific symptom of a disease, or an antibody against them.

7. A reaction detecting method according to claim 6, wherein the specific substance is a cancer-related substance including a carcinoembryonic protein, a hormone, a hormone receptor, a membrane antigen and a cancer-related gene products; or an antibody against these substances.

8. A reaction detecting method according to claim 5, 6 or 7, wherein the sample is a body fluid including blood, serum, plasma, urine, ascites; a tissue or a tissue extract; or a cell or a cellular extract.

9. A reaction detecting method according to any one of claims 1 to 8, further comprising measuring a temperature of the electrolytic solution, and conducting a temperature correction of a measured value of electric conductivity.

10. A reaction detecting method according to any one of claims 1 to 8, further comprising adopting any one or a combination of: (1) maintaining an atmosphere outside the reaction system in which the reaction of the substance takes place in the electrolytic solution at a constant temperature; (2) thermally shielding the reaction system from the external atmosphere; and (3) maintaining the reaction system and the external atmosphere at the same temperature, without conducting a temperature correction of a measured value of electric conductivity.

11. A reaction detecting method according to any one of claims 2 to 8, further comprising:

adopting any one or a combination of: (1) maintaining an atmosphere outside the reaction system in which the reaction of the substance takes place in the electrolytic solution at a constant temperature; (2) thermally shielding the reaction system from the external atmosphere; and (3) maintaining the reaction system and the external atmosphere at the same temperature, without conducting a temperature correction of a measured value of electric conductivity, and detecting the reaction status, an antigen and/ or the reaction product of the reaction of the substance in the electrolytic solution by time-dependently measuring a temperature of the electrolytic solution and measuring an amount of change or a rate of change in electric conductivity per unit temperature.

12. An immune reaction detecting method comprising detecting an immune reaction between an antigen and an antibody in an electrolytic solution on the basis of measurement of an electric conductivity of the electrolytic solution.

13. An immune reaction detecting method according to claim 12, wherein a reaction status of the immune reaction, the antigen, the antibody and/or an immune complex are detected by time-dependently measuring the electric conductivity of the electrolytic solution.

14. An immune reaction detecting method according to claim 12 or 13, further comprising measuring a temperature of the electrolytic solution, and conducting a temperature correction of a measured value of electric conductivity.

15. An immune reaction detecting method according to claim 12 or 13, further comprising adopting any one or a combination of: (1) maintaining an atmosphere outside the reaction system in which the immune reaction between the antigen and the antibody takes place in the electrolytic solution at a constant temperature; (2) thermally shielding the reaction system from the external atmosphere; and (3) maintaining the reaction system and the external atmosphere at the same temperature, without conducting a temperature correction of a measured value of electric conductivity.

16. An immune reaction detecting method according to claim 13, further comprising:

adopting any one or a combination of: (1) maintaining an atmosphere outside the reaction system in which the reaction of the substance takes place in the electrolytic solution at a constant temperature; (2) thermally shielding the reaction system from the external atmosphere; and (3) maintaining the reaction system and the external atmosphere at the same temperature, without conducting a temperature correction of a measured value of electric conductivity, and detecting the reaction status and/or the reaction product of the reaction of the substance in the electrolytic solution by time-dependently measuring a temperature of the electrolytic solution and measuring an amount of change or a rate of change in electric conductivity per unit temperature.

17. An immune reaction detecting method comprising detecting an immune reaction between an antigen and an antibody in a subject solution on the basis of measurement of a temperature of the subject solution.

18. An immune reaction detecting method according to claim 17, wherein a reaction status of the immune reaction, the antigen, the antibody and/or an immune complex by time-dependently measuring the temperature of the subject solution.

19. An immune reaction detecting method according to claim 17, further comprising adopting any one or a combination of: (1) maintaining an atmosphere outside the reaction system in which the immune reaction between the antigen and the antibody takes place in the subject solution at a constant temperature; (2) thermally shielding the reaction system from the external atmosphere; and (3) maintaining the reaction system and the external atmosphere at the same temperature to measure the temperature of the subject solution.

20. An immune reaction detecting method according to any one of claims 12 to 19, wherein the antigen is a substance associated with a specific symptom of a disease or a gene product associated with a specific state of a disease, or the antibody is a substance associated with a specific symptom of a disease or an antibody against a gene product associated with a specific state of disease; and the antigen or the antibody is detected and/or quantitatively determined by detecting the immune reaction.

21. An immune reaction detecting method according to any one of claims 12 to 19, wherein the antigen is a cancer-related substance including a carcinoembryonic protein, a hormone, a hormone receptor, a membrane antigen and a cancer-related gene product, or the antibody is an antibody against a cancer-related substance including a carcinoembryonic protein, a hormone, a hormone receptor, a membrane antigen, and a cancer-related gene product; and the antigen or the antibody is detected and/or quantitatively determined by detecting the immune reaction.

22. A reaction detecting apparatus comprising:

a reactor for containing a reactant and an electrolytic solution;
electric conductivity detecting means issuing a signal corresponding to an electric conductivity of the electrolytic solution in the reactor; and
control means detecting a signal issued by the electric conductivity detecting means in response to a reaction of a substance in the electrolytic solution.

23. A reaction detecting apparatus according to claim 22, further comprising temperature detecting means issuing a signal corresponding to a temperature of the electrolytic solution in the reactor to correct a measured value of electric conductivity based on an output of the electric conductivity detecting means on the basis of an output of the temperature detecting means.

24. A reaction detecting apparatus according to claim 22, further comprising any one or a combination of: (1) means for maintaining an atmosphere outside the reactor at a constant temperature; (2) means for thermally shielding the interior of the reactor from the atmosphere outside the reactor; and (3) means for maintaining the interior of the reactor and atmosphere outside the reactor at the same temperature.

25. A reaction detecting apparatus according to claim 24, further comprising temperature detecting means issuing a signal corresponding to a temperature of the electrolytic solution in the reactor, wherein the control means generates a signal corresponding to an amount of change or a rate of change in electric conductivity per unit temperature on the basis of a signal issued by the electric conductivity detecting means in response to the reaction of the substance in the electrolytic solution, and a signal issued by the temperature detecting means in response to the reaction of the substance in the electrolytic solution.

26. An immune reaction detecting apparatus comprising:

a reactor for containing a subject solution containing an antigen and an antibody; electric conductivity detecting means issuing a signal corresponding to an electric conductivity of the electrolytic solution in the reactor, and control means detecting a signal issued by the electric conductivity detecting means in response to an immune reaction between the antigen and the antibody in the electrolytic solution.

27. An immune reaction detecting apparatus according to claim 26, further comprising temperature detecting means issuing a signal corresponding to a temperature of the electrolytic solution in the reactor to correct a measured value of electric conductivity based on an output of the electric conductivity detecting means on the basis of an output of the temperature detecting means.

28. An immune reaction detecting apparatus according to claim 26, further comprising any one or a combination of: (1) means for maintaining an atmosphere outside the reactor at a constant temperature; (2) means for thermally shielding the interior of the reactor from the atmosphere outside the reactor, and (3) mains for maintaining the interior of the reactor and the atmosphere outside the reactor at the same temperature.

29. An immune reaction detecting apparatus according to claim 28, further comprising temperature detecting means issuing a signal corresponding to a temperature of the electrolytic solution is the reactor, wherein the control means generates a signal corresponding to an amount of change or a rate of change in electric conductivity per unit temperature on the basis of a signal issued by the electric conductivity detecting means in response to the immune reaction between the antigen and the antibody in the electrolytic solution, and a signal issued by the temperature detecting means in response to the immune reaction between the antigen and the antibody in the electrolytic solution.

30. An immune reaction detecting apparatus comprising:

a reactor for containing a subject solution containing an antigen and an antibody; temperature detecting means issuing a signal corresponding to a temperature of the subject solution in the reactor; and control means detecting a signal issued by the temperature detecting means in response to an immune reaction between the antigen and the antibody in the subject solution.

31. An immune reaction detecting apparatus according to claim 30, further comprising any one or a combination of: (1) means for maintaining an atmosphere outside the reactor at a constant temperature; (2) means for thermally shielding the interior of the reactor from the atmosphere outside the reactor; and (3) means for maintaining the interior of the reactor and the atmosphere outside the reactor at the same temperature.

# FIG.1A

# FIG.1B

FIG.2

# FIG.3

CHANGE IN ELECTRIC CONDUCTIVITY IN REACTION BETWEEN K1
ANTIBODY (0.5ng) AND CEA ANTIGEN

EP 1 319 949 A1

FIG.4

CHANGE IN ELECTRIC CONDUCTIVITY WITH K1 ANTIBODY OR
BFP STANDARD ANTIGEN ALONE

EP 1 319 949 A1

# FIG.5

CHANGE IN ELECTRIC CONDUCTIVITY IN REACTION BETWEEN K1
ANTIBODY (0.5ng) AND STANDARD BFP ANTIGEN

EP 1 319 949 A1

## FIG.6

CHANGE IN ELECTRIC CONDUCTIVITY AT POINTS IN REACTION
TIME LAPSE IN REACTION BETWEEN K1 ANTIBODY AND BFP ANTIGEN

# FIG.7

CHANGE IN ELECTRIC CONDUCTIVITY IN REACTION BETWEEN K1
ANTIBODY (0.5ng) AND SUBJECT SERUM

EP 1 319 949 A1

## FIG.8

CHANGE IN ELECTRIC CONDUCTIVITY IN REACTION BETWEEN
MDM2 ANTIGEN (200ng) AND MDM2 ANTIBODY

EP 1 319 949 A1

# FIG.9

CHANGE IN ELECTRIC CONDUCTIVITY AT POINTS IN REACTION
TIME LAPSE IN REACTION BETWEEN MDM2 ANTIGEN AND MDM2 ANTIBODY

# FIG.10

CHANGE IN ELECTRIC CONDUCTIVITY IN REACTION BETWEEN
MDM2 ANTIGEN (200ng) AND SUBJECT SERUMS

EP 1 319 949 A1

EP 1 319 949 A1

FIG.11

CHANGE IN ELECTRIC CONDUCTIVITY IN REACTION BETWEEN K1
ANTIBODY (50ng) AND PEPSIN (17.6U/5 μg)

36

# FIG.12

CHANGE IN ELECTRIC CONDUCTIVITY IN CHANGE
IN TEMPERATURE OF PHYSIOLOGICAL SALINE
(WITHOUT TEMPERATURE CORRECTION)

# FIG.13

AMOUNT OF CHANGE IN ELECTRIC CONDUCTIVITY
PER 1°C OF SOLUTION TEMPERATURE AND
TEMPERATURE COEFFICIENT

# FIG.14

CHANGE IN ELECTRIC CONDUCTIVITY AND SOLUTION
TEMPERATURE IN REACTION BETWEEN BFP
ANTIGEN AND K1 ANTIBODY (0.5ng)
(WITHOUT TEMPERATURE CORRECTION)

EP 1 319 949 A1

FIG.15A

AMOUNT OF CHANGE IN ELECTRIC CONDUCTIVITY PER 1℃
OF SOLUTION TEMPERATURE IN REACTION BETWEEN BFP ANTIGEN
AND K1 ANTIBODY (0.5ng) (WITHOUT TEMPERATURE CORRECTION)

FIG.15B

TEMPERATURE COEFFICIENT (%) IN REACTION BETWEEN
BFP ANTIGEN AND K1 ANTIBODY (0.5ng)
(WITHOUT TEMPERATURE CORRECTION)

40

EP 1 319 949 A1

FIG.16A

CHANGE IN ELECTRIC CONDUCTIVITY AND SOLUTION
TEMPERATURE WITH K1 ANTIBODY (1ng) ALONE
(WITHOUT TEMPERATURE CORRECTION)

FIG.16B

CHANGE IN ELECTRIC CONDUCTIVITY AND SOLUTION
TEMPERATURE WITH BFP ANTIGEN (4.8ng) ALONE
(WITHOUT TEMPERATURE CORRECTION)

41

# FIG.17

CHANGE IN ELECTRIC CONDUCTIVITY AND SOLUTION
TEMPERATURE IN REACTION BETWEEN BFP ANTIGEN
AND K1 ANTIBODY (0.5ng) UPON ADDING FCS
(WITHOUT TEMPERATURE CORRECTION)

EP 1 319 949 A1

FIG.18A

AMOUNT OF CHANGE IN ELECTRIC CONDUCTIVITY PER 1°C
OF SOLUTION TEMPERATURE IN REACTION BETWEEN
BFP ANTIGEN AND K1 ANTIBODY (0.5ng)
(FCS ADDED, WITHOUT TEMPERATURE CORRECTION)

FIG.18B

TEMPERATURE COEFFICIENT (%) IN REACTION
BETWEEN BFP ANTIGEN AND K1 ANTIBODY (0.5ng)
(FCS ADDED, WITHOUT TEMPERATURE CORRECTION)

FIG.19A

CHANGE IN ELECTRIC CONDUCTIVITY AND SOLUTION
TEMPERATURE IN REACTION BETWEEN HEALTHY
PERSON SERUM AND K1 ANTIBODY (0.5ng)
(WITHOUT TEMPERATURE CORRECTION)

FIG.19B

CHANGE IN ELECTRIC CONDUCTIVITY AND SOLUTION
TEMPERATURE IN REACTION BETWEEN PATIENT
SERUM AND K1 ANTIBODY (0.5ng)
(WITHOUT TEMPERATURE CORRECTION)

## FIG.20A

AMOUNT OF CHANGE IN ELECTRIC CONDUCTIVITY AND
TEMPERATURE COEFFICIENT IN REACTION BETWEEN
HEALTHY PERSON SERUM AND K1 ANTIBODY (0.5ng)
(WITHOUT TEMPERATURE CORRECTION)

## FIG.20B

AMOUNT OF CHANGE IN ELECTRIC CONDUCTIVITY AND
TEMPERATURE COEFFICIENT IN REACTION BETWEEN
PATIENT SERUM AND K1 ANTIBODY (0.5ng)
(WITHOUT TEMPERATURE CORRECTION)

# FIG.21

CHANGE IN ELECTRIC CONDUCTIVITY IN REACTION
BETWEEN PATIENT SERUM AND K1 ANTIBODY
(WITHOUT TEMPERATURE CORRECTION)

EP 1 319 949 A1

FIG.22A

CHANGE IN ELECTRIC CONDUCTIVITY AND SOLUTION
TEMPERATURE IN REACTION BETWEEN BFP
ANTIGEN AND K1 ANTIBODY (0.5ng)
(WITHOUT TEMPERATURE CORRECTION)

FIG.22B

CHANGE IN ELECTRIC CONDUCTIVITY AND SOLUTION
TEMPERATURE IN REACTION BETWEEN BFP
ANTIGEN AND K1 ANTIBODY (0.5ng)
(FCS ADDED, WITHOUT TEMPERATURE CORRECTION)

## FIG.23

# FIG.24

1

DISPLAY MEANS 14

CONTROL MEANS 11

INPUT MEANS 13

2

10

MEMORY MEANS 12

3

20

S

# FIG.25

100

DISPLAY MEANS 14

CONTROL MEANS 11

INPUT MEANS 13

MEMORY MEANS 12

10

3

20

S

## INTERNATIONAL SEARCH REPORT

International application No.

PCT/JP01/08188

A. CLASSIFICATION OF SUBJECT MATTER
Int.Cl⁷ G01N33/483, 33/53, 27/06

According to International Patent Classification (IPC) or to both national classification and IPC

B. FIELDS SEARCHED

Minimum documentation searched (classification system followed by classification symbols)
Int.Cl⁷ G01N33/483, 33/53, 27/06

Documentation searched other than minimum documentation to the extent that such documents are included in the fields searched
Jitsuyo Shinan Koho      1922-1996    Toroku Jitsuyo Shinan Koho  1994-2001
Kokai Jitsuyo Shinan Koho  1971-2001    Jitsuyo Shinan Toroku Koho  1996-2001

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 94/23287 A (DKK Corporation), 13 October, 1994 (13.10.94), & AU 6292494 A | 1-16,20-29 |
| Y | Microfilm of the specification and drawings annexed to the request of Japanese Utility Model Application No. 198361/1981 (Laid-open No. 103361/1983), (Kabushiki Kaisha Hokushin Denki Seisakusho), 14 July, 1983 (14.07.83) | 1-16,20-29 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

Date of the actual completion of the international search
05 December, 2001 (05.12.01)

Date of mailing of the international search report
18 December, 2001 (18.12.01)

Name and mailing address of the ISA/
Japanese Patent Office

Authorized officer

Facsimile No.

Telephone No.

Form PCT/ISA/210 (second sheet) (July 1992)